# EUROPÄISCHE PATENTSCHRIFT

(11) **EP 2 318 060 B1**
(45) Veröffentlichungstag und Bekanntmachung des Hinweises auf die Patenterteilung: **12.12.2012**
(21) Anmeldenummer: 09781383.6
(22) Anmeldetag: 31.07.2009
(51) Int. Cl.: A61L 27/34, A61L 29/08, A61L 31/10, C23C 16/30, C23C 16/42

(54) **BIOKOMPATIBILITÄTSSCHICHT UND BESCHICHTETE GEGENSTÄNDE**
BIOCOMPATIBILITY LAYER, AND COATED OBJECTS
COUCHE DE BIOCOMPATIBILITÉ ET OBJETS REVÊTUS

(30) Priorität: 31.07.2008 EP 08161598
(43) Veröffentlichungstag der Anmeldung: 11.05.2011
(73) Patentinhaber: Fraunhofer-Gesellschaft zur Förderung der angewandten Forschung e.V., 80686 München (DE); Bio-Gate AG, 28359 Bremen (DE)
(72) Erfinder: OTT, Matthias, 21255 Dohren (DE); GRUNWALD, Ingo, 28205 Bremen (DE); SALZ, Dirk, 28195 Bremen (DE); WAGENER, Michael, 28355 Bremen (DE); VISSING, Klaus-Dieter, 27321 Thedinghausen (Morsum) (DE); HIELSCHER, Wolfgang, 28755 Bremen (DE); DÖLLE, Christopher, 27749 Delmenhorst (DE)
(74) Vertreter: Eisenführ, Speiser & Partner
(86) Internationale Anmeldenummer: PCT/EP2009/059985
(87) Internationale Veröffentlichungsnummer: WO 2010/012836

(56) Entgegenhaltungen:
- WO-A-2008/074388
- DE-A1-102006 018 491
- US-A- 4 508 606

## Beschreibung

Die vorliegende Erfindung betrifft die Verwendung einer vernetzten siliziumhaltigen Schicht enthaltend, bestehend im wesentlichen aus oder bestehend aus Silizium, O, C, H, optional N, die durch Plasmapolymerisation und/oder Vernetzung von siliziumorganischen Flüssigkeiten mit Hilfe eines Plasmaprozesses und/oder UV-Strahlung einer Wellenlänge unter 250 nm ohne die Verwendung von Metallen einer Ordnungszahl über 14 herstellbar ist als nicht-genotoxische Oberfläche oder zum Vermitteln oder Versehen einer Oberfläche mit einer nicht-genotoxischen Wirkung., Außerdem betrifft die Erfindung die Verwendung der vorbeschriebenen Schichten zur definierten i) Verbesserung oder ii) Herabsetzung der Adhäsion von Biomolekülen und/oder Zellen (Pro oder Eukaryonten). Ferner betrifft die Erfindung entsprechend beschichtete Gegenstände und Verfahren zu ihrer Herstellung.

Nicht-Genotoxizität im Sinne der vorliegenden Erfindung bezeichnet allgemein die Eigenschaft eines Materials, keine oder eine lediglich akzeptable Genotoxizität gemessen gemäß ISO 10993-3:2003 auszuüben. Dabei wird das Vorliegen der Eigenschaft "Nicht-Genotoxizität" bevorzugt gemäß dem in der ISO 10993-3:2003 ermittelten Verfahren ermittelt. Alternativ (oder ergänzend) ist auch das in dieser Anmeldung weiter unten in Beispiel 5 beschriebene Verfahren geeignet, "in Anlehnung an die DIN IN ISO 10993-3" das Vorliegen von Nicht-Genotoxizität im Sinne der vorliegenden Anmeldung zu belegen.

Die Eigenschaft von Gegenständen nicht-genotoxisch zu sein, stellt insbesondere im Medizinbereich eine besonders hohe Anforderung dar: Genotoxizität zeigt ihre Auswirkung zum Teil erst nach einem langen Zeitraum. Dabei können kleinste Mengen einer genotoxischen Substanz, sofern sie über längere Zeit im Körper verbleibt, langfristige und ggf. für einen Organismus tödliche Auswirkungen haben. Im Medizinbereich ist Nicht-Genotoxizität in Abhängigkeit von der Anwendung nicht immer eine Voraussetzung für eine ausreichende Biokompatibilität, da Biokompatibilität auch eine Biotoleranz für bestimmte Zeiträume umfasst, die kürzer sein können als die Wirkzeiträume eines potentiell genotoxischen Stoffes.

Eine nicht-genotoxische Schicht im Sinne der vorliegenden Erfindung ist dementsprechend eine Schicht, die, wenn sie in unmittelbaren Kontakt mit lebenden Zellen, Geweben, Organen oder Lebewesen, insbesondere einem Menschen oder einer menschlichen Zelle, Gewebe oder Organ steht, Nicht-Genotoxizität gemäß der oben beschriebenen Definition aufweist. Erfindungsgemäß bilden nicht-genotoxische Schichten vorzugsweise Oberflächen auf Gegenständen, die bei bestimmungsgemäßer Anwendung in Berührung mit menschlichen Zellen, Gewebe oder Organen gebracht werden, wobei die in Berührung mit den Zellen, Gewebe oder Organen stehende Oberfläche des Gegenstandes zumindest teilweise mit einer nicht-genotoxische Schicht bedeckt ist. Gegenstände, die bei bestimmungsgemäßer Anwendung in Berührung mit menschlichen Zellen, Gewebe oder Organen gebracht werden, werden im Rahmen dieser Erfindung auch vereinfacht als Medizinprodukt bezeichnet.

Verschiedene Gegenstände mit biokompatibler (und teilweise auch nicht-genotoxischer) Oberfläche sind als solche bereits bekannt. Wie nachfolgend dargelegt war es bisher jedoch aufwändig, entsprechende Oberflächen auf diesen Gegenständen herzustellen, oder Verfahren, die bei einem bestimmten Gegenstand zur Ausbildung einer solchen Oberfläche führen, auf andere Gegenstände zu übertragen:

In verschiedenen technischen Bereichen wie der Sensortechnologie, insbesondere aber auch im Bereich von Medizinprodukten, beispielsweise bei Implantaten, Stents, Schrittmachern, Injektionssystemen oder Kathetern, spielt die Kompatibilität der verwendeten Materialien eine wichtige Rolle. Neben der generellen guten Verträglichkeit solcher Gegenstände werden fast immer auch noch zusätzliche Eigenschaften in den jeweiligen Einsatzgebieten gefordert. Um diese Eigenschaften zu erzielen, werden herkömmlicherweise deren Oberflächen durch (nass)chemische und/oder physikalische Verfahren bearbeitet. Hierdurch soll erreicht werden, dass sich zum Beispiel Proteine wie beispielsweise Albumin und Fibronectin an den behandelten Oberflächen besser anlagern. Ein Beispiel einer solchen Oberflächenbehandlung ist das Aufbringen geeigneter Oberflächengruppen wie z.B. Amino-, Hydroxyl- oder Carboxylgruppen. Das hierbei verfolgte Ziel ist, dass die jeweiligen behandelten Gegenstände (beispielsweise Implantate) sich leichter in vorhandene Gewebestrukturen einpassen können, indem sie beispielsweise im Falle von Implantaten fester mit Knochengewebe verbunden werden und diese Verbindung verträglicher ausfällt, insbesondere indem lokale pathologische Effekte in lokalem Gewebe auf makroskopischer und mikroskopischer Ebene begrenzt oder verringert werden. Durch eine Oberflächenbearbeitung soll in anderen Fällen auch erreicht werden, dass (Körper-) Zellen, insbesondere Knochenzellen, Gewebezellen, Endothelzellen, sich an diese Oberflächen anlagern und diese bewachsen, so dass es zu einer schnelleren Integration des Gegenstandes, z.B. des Implantats, in das umliegende Gewebe kommt. Dies ist zum einem für eine verbesserte Verankerung und Verträglichkeit wünschenswert. Zum anderen wird hierdurch die Heilung beschleunigt und das Risiko einer entzündlichen Abstoßung reduziert.

Auf der anderen Seite versucht man, bei manchen Gegenständen genau den gegenteiligen Effekt zu erreichen, also die Anlagerung von Biomolekülen und Zellen auf bestimmten Oberflächen zu vermeiden. Durch eine Anlagerung kann es zu einer unerwünschten Funktionseinschränkung z.B. im Bereich von Sensoren, aber auch zum Verstopfen von Kanülen oder Schläuchen kommen. Diese Effekte werden auch als Fouling bezeichnet. Implantate haben häufig Zonen, in denen ein Zellbewuchs unerwünscht ist. Bei Kurzzeit-Kathetern kann durch eine zu gute Gewebeintegration die einfache Entfernung erschwert werden.

Ein weiterer nachteiliger Effekt von gut mit Biomolekülen und Zellen verbindbaren Oberflächen besteht darin, dass durch eine unerwünschte Interaktion von Zellen mit den eingebrachten Materialien zellbiologische Reaktionen wie beispielsweise Entzündungsreaktionen und Zellagglomerationen induziert werden können, welche zu schwersten Nebenwirkungen führen können.

Die Besiedlung von Implantatoberflächen oder Prothesen mit Mikroorganismen, z.B. durch Antibiotika-resistente Staphylokokken, stellt ein schwerwiegendes Problem in der Medizin dar. Durch die Infektion der Patienten, respektive die Besiedlung der Implantate, kann es zu lebensbedrohenden Nebenwirkungen kommen. Diese sind oftmals nur durch einen sehr kostspieligen Ersatz des Implantates und einen langen Krankenhausaufenthalt zu begegnen. Der Bakterienadhäsion geht immer eine Adhäsion von Biomolekülen wie z.B. Proteinen aus dem Serum voraus. Geeignete anti-adhäsive Beschichtungen können hierbei die Besiedlung mit Bakterien stark reduzieren.

Zur Behandlung von Krankheiten, wie z.B. Infektionen, werden dem Patienten häufig Infusionen verabreicht oder Zugänge gelegt. Hierbei wird dem Patienten meist ein Katheter, ein Port oder eine Kanüle gesetzt. Im Bereich des Durchtritts bzw. im Körper kommt es häufig zu entzündlichen Reaktionen, die u.a. durch das eingesetzte Material hervorgerufen werden. Eine die Zelladhäsion fördernde Beschichtung vermag hier diese unerwünschten Reaktionen zu reduzieren.

Für die Behandlung schwerster Krankheiten, beispielsweise aggressive Tumore, Autoimmunerkrankungen oder bei Infektionen, stehen seit einigen Jahren neue Protein - und DNA-basierte Medikamente zur Verfügung. Hierbei handelt es sich z.B. um Antiköper gegen Tumorzellen, Peptidantibiotika gegen Infektionen oder siRNA gegen Autoimmunerkrankungen. Der Vorteil dieser neuen Wirkstoffe liegt u.a. darin, dass diese in relativ geringen Mengen wirksam sind. Hierbei tritt jedoch das Problem auf, dass bei der Verabreichung (z.B. intravenös) diese Substanzen an die Oberflächen der Spritzen, Schläuche oder Vorratsflaschen adsorbieren, sich also unspezifisch und ungewollt anlagern. Um den Wirkstoffverlust zu kompensieren, werden diese meist 15- 25 % höher dosiert als dies eigentlich notwendig wäre.

Eine weitere Aufgabe der Beschichtung ist es, eine nicht schädigende Interaktion einer Hohlfaser von Membran-Oxygenatoren mit Blut zu ermöglichen. Ein Oxygenator ist ein Teil einer Herz-Lungen-Maschine, die dem Blut Sauerstoff zuführt. Der Oxygenator umfasst Hohlfasern, in denen Sauerstoff hindurch geführt wird. An der äußeren Oberfläche der Fasern läuft der Blutstrom entlang. Dieser sogenannte Membran-Oxygenator tauscht Kohlendioxid gegen Sauerstoff aus.

Herkömmlicherweise sind zahlreiche Versuche unternommen worden, Oberflächen mit Fouling-verhindernden oder -verzögernden Eigenschaften (sogenannte "Anti-Fouling-Oberflächen") und/oder mit anti-adhäsiven Eigenschaften zu versehen. Einige dieser Versuche werden nachfolgend kurz beschrieben. Dabei werden auch solche Dokumente gewürdigt, deren Inhalt erst rückschauend, also in Kenntnis der fertigen vorliegenden Erfindung, als strukturell zumindest entfernt ähnlich erkannt wurde, auch wenn sie selbst keinen Hinweis zur Lösung der Aufgabe der Erfindung enthalten.

Dem Stand der Technik entsprechend können z.B. Implantatoberflächen durch nasschemische Verfahren mit Polyethylenglykol (PEG), Poly(ethylenoxid) (PEO) oder ähnlichen Polymeren beschichtet werden, um anti-fouling oder anti-adhäsive Oberflächen herzustellen.

Es ist bekannt, dass mit CHF₃-Pulsplasmen Oberflächen derartig beschichtet werden können, dass das Anhaften (Adhäsion) und das Wachstum von Zellen zu beeinflussen ist (M. Haupt, J. Barz, U. Vohrer, H. Hilgers, C. Oehr in Vakuum in Forschung und Praxis, 2005, Band 17, Ausgabe 6, Seiten 329 - 335). Ein derartiges Vorgehen ist aber mit den bekannten Problemen bei der Verwendung von fluorhaltigen Plasmen verbunden: Die Precursoren sind vergleichsweise teuer und können zu cancerogenen Zwischenprodukten führen.

Zudem ist die Abscheidung von speziellen Siliziumcarbid-Plasmabeschichtungen zur Verbesserung der Bio- und Hämokompatibilität von Stentgrundkörpern bekannt (Vortrag von Dr. Carsten Momma zum Themenkomplex" Plasmatechnik in der Gefäßmedizin" auf dem Innovationsforum "PlasmaPlusBio" am 02. Februar 2006 in Greifswald). Hierbei handelt es sich um ein sehr teures Beschichtungsverfahren, da die Beschichtungsanlagen für Hochvakuum ausgestattet sein müssen.

Die US5182317 offenbart ein Beschichtungsverfahren, bei dem eine Oberflächenbeschichtung mit Aminen funktionalisiert wird. Das Verfahren ist verhältnismäßig aufwändig und entsprechend teuer durchzuführen. Ähnlich aufwändige Verfahren sind beispielsweise aus der US5455040 bekannt.

Bei Membran-Oxygenatoren werden gegenwärtig häufig Heparinmoleküle beispielsweise über einen chemischen Prozess an die äußere Oberfläche der Faser angebunden. Jedoch hat diese Methode den Nachteil, dass sie sehr aufwändig ist und der Sauerstoff/Kohlendioxid-Austausch im Blut mit derart behandelten Fasern nur schlecht funktioniert.

Aus DE69932273 T2 ist ferner bekannt, dass Oxygenatorfasern mit Aminfunktionalitäten versehen werden können. Dieses geschieht durch Auftragung einer aminfunktionellen Polysiloxanlösung. Anschließend bringt man die so funktionalisierte Oberfläche mit einem Biomolekül in Kontakt, beispielsweise mit Heparin. Heparin soll die Bildung von Thromben verhindern, so dass die Thrombosegefahr für den Patienten sinkt. Kommerziell werden Oxygenatoren mit dieser Beschichtung unter dem Markennamen "QUADROX Bioline coated" vertrieben.

Alternativ wird mit Hilfe von Polypeptiden die Oberfläche der Oxygenatorfasern biokompatibel ausgestattet. Diese werden unter dem Handelsnamen "QUADROX Safeline treated" vertrieben.

Die US6549811 offenbart die Verwendung plasmapolymerer Beschichtungen zur Strukturierung der Oberflächen von Elastomeren wie Kathetern aus Silikon.

Die EP1060031 B1 beschreibt die Vernetzung von Silikonöl mit Plasma als Beschichtung für einen Behälter für ein pharmazeutisches Proteinpräparat. Die Beschichtung soll die Adhäsion von Proteinen an der beschichteten Oberfläche verringern.

Die WO 2005/014075 A1 offenbart allgemein eine Plasmapolymerisation mit Siloxanen zur Beschichtung von "Ausdehnungballons" zum Verbessern der Abrasionsbeständigkeit und zum Vorbeugen gegen kleinste Löcher.

Aus der EP1701676B1 ist eine Wundauflage bekannt, die eine antimikrobiell wirksame Substanz (Silber) in einem durch Plasmapolymerisation aus Hexamethyldisiloxan gebildeten Polymer besitzt. Über einen Anti-Fouling-Effekt oder anti-adhäsive Eigenschaften einer silberfreien Oberfläche ist nichts angegeben.

Die DE 103 537 56 offenbart ein antimikrobielles, nicht zytotoxisches Schichtmaterial. Das Schichtmaterial enthält Silber und eine plasmapolymere Schicht als Transportkontrollschicht. Die DE 103 537 56 offenbart jedoch nicht, dass auch ohne Anwesenheit von Silber Zytotoxizität vermieden oder gar Nicht-Genotoxizität im erfindungsgemäßen Sinne herbeigeführt werden kann. Ferner erlaubt das beschriebene Verfahren nicht bzw. nur sehr aufwändig die Beschichtung komplexer Bauteilgeometrien.

Gleichfalls zeigt die US6589546 allgemein ein Wirkstoffabgabesystem mit plasmapolymerer Deckschicht, jedoch ohne nähere Erläuterungen zu Wechselwirkungen mit Körperzellen.

Die WO 02100928 A1 offenbart verschiedene plasmapolymere Schichten, ohne jedoch einen Hinweis auf eventuelle Wechselwirkungen mit Körperzellen oder den Einsatz im direkten Kontakt mit Körpergewebe zu geben.

Aus der EP 982041 A1 sind spezielle Plasma-Beschichtungen mit funktionalisierten Siloxanen bekannt. Sie sollen zur Bildung thromboresistenter Beschichtungen medizinischer Geräte dienen. Die vorgestellten Beschichtungen sind aufwändig herzustellen.

Die DE 10031198 A1 zeigt sehr allgemein Plasma-Beschichtungen von medizintechnischen Oberflächen. Die Beschichtungen sollen als Korrosionsschutz wirken. Über eine verbesserte Biokompatibilität ist nichts Konkretes gesagt.

Ferner zeigt die EP 745220 B1 "bio-inerte" Festphasensensoren mit einer aus Silikoncarbinol gebildeten Beschichtung.

Die DE 10 2006 018 491 A1 offenbart plasmapolymere Schichten einer bestimmten Zusammensetzung, die ganz allgemein als "nicht-zytotoxisch" oder "körperverträglichkeitsverbessernd" beschrieben werden. Ferner offenbart dies Dokument, dass diese Schichten auf implantierbaren medizinischen Gegenständen vorgesehen werden können. Dies ist jedoch kein Hinweis auf ein Vorliegen oder Ermöglichen einer Nicht-Genotoxizität im Sinne der vorliegenden Anmeldung: Zum einen ist die Art der implantierbaren Gegenstände nicht angegeben, so dass nicht deutlich ist, ob diese nur kurzfristig in den Körper implantiert werden oder über sehr lange Zeiträume dort verbleiben sollen. Zum anderen ist lediglich von einer Verbesserung der Körperverträglichkeit in dem entsprechenden Dokument im Zusammenhang mit implantierbaren medizinischen Gegenständen im Sinne einer Verminderung der Haftung von Bakterien, Eiweißen oder anderen körpereigenen Stoffen die Rede, was aber ebenfalls keinen Hinweis auf das Vorliegen von Nicht-Genotoxizität beinhaltet.

Aufgabe der vorliegenden Erfindung war es daher, den Nachteilen des Standes der Technik abzuhelfen oder diese Nachteile zu mindern und verbesserte Beschichtungen sowie entsprechend beschichtete Gegenstände und Verwendungen der Beschichtungen anzugeben. Insbesondere sollten dem Fachmann Möglichkeiten eröffnet werden, medizinische Artikel auf kostengünstige Weise und effektiv mit Oberflächeneigenschaften auszustatten, die vorteilhaft für den Einsatz im Zusammenhang mit der Berührung von Teilen des Körpers sind. Besonderes Augenmerk sollte in diesem Zusammenhang darauf gelegt werden, dass auch langfristige Berührungen auch über einen langen Zeitraum keine schädigende Wirkung im Körper entfalten. Solche langfristigen Berührungen können erzeugt werden z.B. durch ein dauerhaftes Implantat oder durch Abriebreste (auch in kleinsten Mengen), die auch nur bei temporärer (kurzzeitiger) Berührung mit dem Körper entstehen und im ungünstigen Falle dauerhaft mit diesem in Kontakt bleiben können (indem sie z.B. eingebaut werden).

Die Aufgabe wird gelöst durch Angabe einer Verwendung einer vernetzten siliziumhaltigen Schicht enthaltend, bestehend im wesentlichen aus oder bestehend aus Silizium, O, C, H, optional N, die durch Plasmapolymerisation und/oder Vernetzung von siliziumorganischen Flüssigkeiten mit Hilfe eines Plasmaprozesses und/oder UV-Strahlung einer Wellenlänge unter 250 nm ohne die Verwendung von Metallen einer Ordnungszahl über 14 herstellbar ist, mit einem atomaren Verhältnis von Sauerstoff zu Silizium von 0,75 bis 2,2 und einem atomaren Verhältnis von Kohlenstoff zu Silizium von 0,1 bis 2,5, gemessen mit XPS,
- als nicht-genotoxische Oberfläche oder
- zum Vermitteln oder Versehen einer Oberfläche mit einer nicht-genotoxischen Wirkung.

Insbesondere wird eine nicht-genotoxische Wirkung erfindungsgemäß vermittelt oder modifiziert durch definierte Beeinflussung der chemischen Zusammensetzung der Oberfläche der siliziumhaltigen Schicht. Auf diese Weise kann auch die Adhäsion von Biomolekülen und/oder pro- und/oder eukaryontischen Zellen definiert eingestellt werden (vgl. auch weiter unten).

Unter einer nicht-genotoxischen Schicht im Sinne der Erfindung wird daher insbesondere eine siliziumhaltige Schicht verstanden, enthaltend, bestehend im wesentlichen aus oder bestehend aus Silizium, O, C, H, optional N, die durch Plasmapolymerisation und/oder Vernetzung von siliziumorganischen Flüssigkeiten mit Hilfe eines Plasmaprozesses und/oder UV-Strahlung einer Wellenlänge unter 250 nm ohne die Verwendung von Metallen einer Ordnungszahl über 14 herstellbar ist.

Die Verwendung einer erfindungsgemäßen Nicht-genotoxischen Schicht erlaubt es insbesondere, die Nicht-Genotoxizität eines entsprechend beschichteten Gegenstandes wie eingangs beschrieben zu gewährleisten. Erfindungsgemäß zu verwendende Nicht-genotoxischen Schichten können leicht hergestellt werden, durch Verwendung geeigneter Precursoren können die Herstellungskosten gering gehalten werden, die Nicht-genotoxischen Schichten können sehr dünn und dennoch voll wirksam hergestellt werden, die Adhäsion von Zellen, Proteinen, Nukleinsäuren und Fettsäuren kann auf einfache Weise eingestellt werden. Die Herstellung entsprechender Schichten kann als reiner Gasphasenprozess im Unterdruck durchgeführt werden, so dass eine Gefahr der Abgabe von nicht reagierten Monomeren praktisch vollständig vermieden werden kann. Erfindungsgemäß zu verwendende Schichten können, wie nachfolgend beschrieben, auf einer Vielzahl unterschiedlicher fester Substrate aufgebracht werden, wobei auch kompliziert geformte Oberflächen gleichmäßig mit der nicht-genotoxischen Schicht versehen werden können. Diese und andere Vorteile der erfindungsgemäß zu verwendenden nicht-genotoxischen Schicht und entsprechend beschichteter Gegenstände werden nachfolgend näher dargestellt.

Durch die erfindungsgemäße Verwendung ist es reproduzierbar und mit überraschend geringem Aufwand möglich, für eine Vielzahl von Gegenständen und Oberflächen-Basismaterialien sowie für verschiedenste Anwendungsgebiete ein einheitliches Material zum Vermitteln nicht-genotoxischer Eigenschaften bereitzustellen. Dabei ist das Erreichen der Nicht-Genotoxizität im Sinne der vorliegenden Anmeldung ein überraschender Effekt: Bei den Herstellungsprozessen für die erfindungsgemäß zu verwendende Schicht entstehen stets eine Vielzahl von sehr reaktiven Spezies (Radikale, geladene Teilchen, jeweils unterschiedlicher im Prinzip nicht genau definierbarer Größe). Solche reaktiven Spezies sind stets potentielle Auslöser von Genotoxizität. Daher konnte der Effekt, dass die erfindungsgemäß zu verwendenden Schichten die Anforderung an fehlende Genotoxizität erfüllen nicht vorhergesehen werden.

Zusätzlich zur Vermittlung nicht-genotoxischer Eigenschaften ermöglicht die erfindungsgemäße Verwendung bevorzugt, einen Gegenstand mit einer Oberfläche zu versehen, die gegenüber einem Menschen und seinen Organen
- keine oder lediglich akzeptable Zytotoxizität, gemessen gemäß ISO 10993-5:2003, insbesondere gemäß Punkt 8.3 der ISO 10993-5:2003, wobei die Kulturen in Kulturmedium mit 10 % fötalem Kälberserum bei 37°C (± 2°C) bei 5% v/v Kohlendioxid inkubiert werden,
- keine oder lediglich akzeptable Reizungen oder intrakutane Reaktivität, gemessen gemäß ISO 10993-10:2003,
- keine oder lediglich akzeptable systemische Toxizität (akute Toxizität, subakute Toxizität und subchronische Toxizität), gemessen gemäß ISO 10993-11:2003,
- ausreichende oder hohe Implantationsverträglichkeit, gemessen gemäß ISO 10993-6:2003, und/oder
- ausreichende oder hohe Hämokompatibilität, gemessen gemäß ISO 10993-4:2003,
aufweist, wobei je nach Einsatzgebiet eines erfindungsgemäß beschichteten Gegenstandes eine oder mehrere der genannten Eigenschaften zusätzlich zur Nicht-Genotoxizität des Gegenstandes erreicht werden können, dazu unten mehr.

Erfindungsgemäß bevorzugt ist eine Verwendung, wobei die erfindungsgemäß einzusetzende Schicht außerdem zur definierten (i) Verbesserung oder (ii) Herabsetzung der Adhäsion von Biomolekülen und/oder Zellen (Pro- oder Eukaryonten) verwendet wird.

Dementsprechend ist ein weiterer Aspekt der Erfindung die Verwendung einer vernetzten siliziumhaltigen Schicht enthaltend, bestehend im wesentlichen aus oder bestehend aus Silizium, O, C, H, optional N, die durch Plasmapolymerisation und/oder Vernetzung von siliziumorganischen Flüssigkeiten mit Hilfe eines Plasmaprozesses und/oder UV-Strahlung einer Wellenlänge unter 250 nm ohne die Verwendung von Metallen einer Ordnungszahl über 14 herstellbar ist, mit einem atomaren Verhältnis von Sauerstoff zu Silizium von 0,75 bis 2,2 und einem atomaren Verhältnis von Kohlenstoff zu Silizium von 0,1 bis 2,5, gemessen mit XPS zur definierten (i) Verbesserung oder (ii) Herabsetzung der Adhäsion von Biomolekülen und/oder Zellen (Pro- oder Eukaryonten) oder die vorbeschriebene bevorzugte erfindungsgemäße Verwendung, jeweils mit der Maßgabe dass für den Fall, dass eine Herabsetzung der Adhäsion durch die Verwendung erfolgt, dass die Schicht keine Schicht ist, bestehend aus Kohlenstoff, Silicium, Sauerstoff und Wasserstoff sowie gegebenenfalls üblichen Verunreinigungen, wobei im ESCA-Spektrum der Schicht, bei Kalibrierung auf den aliphatischen Anteil des C 1s Peaks bei 285,00 eV, im Vergleich mit einem trimethylsiloxy-terminierten Polydimethylsiloxan (PDMS) mit einer kinematischen Viskosität von 350 mm²/s bei 25 °C und einer Dichte von 0,97 g/mL bei 25 °C, der Si 2p Peak einen Bindungsenergiewert besitzt, der um maximal 0,45 eV zu höheren oder niedrigeren Bindungsenergien verschoben ist, und der O 1s Peak einen Bindungsenergiewert besitzt, der um maximal 0,50 eV zu höheren oder niedrigeren Bindungsenergien verschoben ist.

Dabei kann es erfindungsgemäß bevorzugt sein, dass darüber hinaus Schichten ausgeschlossen werden, also zusätzlich insbesondere unter die obige Maßgabe fallen, bei denen der Si 2p Peak einen Bindungsenergiewert besitzt, der um mehr als 0,45 eV zu niedrigeren Bindungsenergien verschoben ist und/oder der O 1s Peak einen Bindungsenergiewert besitzt, der um maximal 0,70 eV zu höheren oder niedrigeren Bindungsenergien verschoben ist.

Wie bereits oben beschrieben ist es mit diesem Aspekt der Erfindung möglich, die Adhäsion für bestimmte Biomoleküle und/oder Zellen auf ein gewünschtes Maß herauf - oder herabzusetzen.

Die oben für die erfindungsgemäße Verwendung ausgeschlossenen Schichten sind nachteilhaft, da ihre Herstellung einen hohen apparativen Aufwand verlangt. Dies liegt darin begründet, dass für den Beschichtungsprozess eine für Niederdruck-Plasmaprozesse vergleichsweise sehr geringe Feuchtigkeit im Prozessgas erlaubt ist. So schreibt die DE 10 2006 018 491 A1, dass zur Herstellung dieser Schichten die Leckrate der benutzten Vakuumkammer deutlich kleiner als 2 mal 10⁻⁹ mbar L pro Sekunde ist. Dies bedeutet höhere Investitionskosten, da eine höhere Anforderung an die Plasmaanlage bezüglich der Dichtigkeit gestellt wird. Darüber hinaus geht für den Fachmann aus den in dem genannten Dokument beschriebenen Beispielen mittelbar hervor, dass eine deutlich verlängerte Abpumpzeit nötig ist, die zu einer Verlängerung der Prozessdauer führen kann. Denn nur so kann bei einer Niederdruck-Technikumsanlage, die ebenso wie übliche Produktionsanlagen Wände aus Edelstahl besitzt und nicht ausgeheizt werden kann, erreicht werden, dass bei hoher Empfindlichkeit des Spektrometers der Messwert für die Masse 18 (Wasser) stark absinkt und dann konstant bleibt. Üblich sind hier Abpumpzeiten von ca. einer Stunde. Da aber Abpumpen annähernd die gleichen Kosten erzeugt, wie das Beschichten selbst, bedeutet eine Verlängerung der Prozessdauer auch eine entsprechende Erhöhung der Beschichtungskosten. In dem Beispiel 1 (vgl. weiter unten) ist belegt, dass bei der dort erzeugten Schicht Plasmapolymer A6 eine nur noch geringe Abpumpzeit möglich ist.

Weiter erfindungsgemäß wird ferner ein nicht-genotoxischer Gegenstand angegeben, umfassend einen Oberflächenbereich mit einer vernetzten siliziumhaltigen Schicht enthaltend, bestehend im wesentlichen aus oder bestehend aus Silizium, O, C, H, optional N, die durch Plasmapolymerisation und/oder Vernetzung von siliziumorganischen Flüssigkeiten mit Hilfe eines Plasmaprozesses und/oder UV-Strahlung einer Wellenlänge unter 250 nm ohne die Verwendung von Metallen einer Ordnungszahl über 14 herstellbar ist, mit einem atomaren Verhältnis von Sauerstoff zu Silizium von 0,75 bis 2,2 und einem atomaren Verhältnis von Kohlenstoff zu Silizium von 0,1 bis 2,5, gemessen mit XPS, wobei der Gegenstand ausgewählt ist aus der Gruppe bestehend aus:
a) Membran, Rohr und Schlauch, insbesondere Oxygenatormembran, Katheter, Angioplasieballon, Stent, Kanüle, Sensor und Sonde,
b) einem bei bestimmungsgemäßem Gebrauch implantierten Gegenstand, ausgewählt aus der Gruppe bestehend aus:
   medizinischer Nagel, Klammer, Faden und Schraube, insbesondere Knochenfixationsnagel, Stent oder Gefäßprothese, Injektionssystem, Katheter, kardiovaskuläres Implantat, künstliches Organ, insbesondere Herzschrittmacher und Stromquelle desselben, Prothese, orthopädisches Implantat, insbesondere künstlicher Gelenkkörper, insbesondere Gelenkpfanne und damit zusammenwirkendes Gegenstück, wie Hüft- oder Knieprothese, Wirbelsäulenprothese, Cochlea-Implantat, künstliche Herzklappe, Herzklappenring oder Intraocularlinse, künstliche Hornhaut, Pumpen oder andere Vorrichtung zur Freisetzung von Substanzen im Körper und Epithese,
c) einem Behältnis zur Aufnahme und/oder zum Transport von Körperflüssigkeit, Gewebe oder deren Bestandteilen eines Lebewesens oder von Biomolekülen, bevorzugt Peptiden, Proteinen, Lipiden, Kohlenhydraten, Nukleinsäuren oder damit hergestellten Wirkstoffen.
d) einem Gegenstand zum zumindest teilweisen Bedecken von Haut oder Schleimhaut eines Lebewesens und bevorzugt von Wunden,
e) einem bei bestimmungsgemäßem Gebrauch anderweitig mit Körperflüssigkeit, Gewebe oder deren Bestandteilen eines Lebewesens oder mit Biomolekülen, bevorzugt Peptiden, Proteinen, Lipiden, Kohlenhydraten, Nukleinsäuren oder damit hergestellten Wirkstoffen in Kontakt stehendem Gegenstand.

Erfindungsgemäß einzusetzende nicht-genotoxische Schichten werden erfindungsgemäß eingesetzt, um auf Gegenständen insbesondere der Medizintechnik definierte Bedingungen bezüglich des Aufwachsens von Körperzellen und/oder der Genotoxizität einzustellen. Dabei kann sowohl ein gutes wie auch ein reduziertes Aufwachsen realisiert werden, wobei die jeweiligen Beschichtungen in beiden Anwendungsbereichen eine nicht-genotoxische Funktion aufzeigen. Parallel zum Aufwachsverhalten von Körperzellen kann die Bakterienadhäsion auf den beschichteten Oberflächen verringert werden. Hierbei konnte gezeigt werden, dass es erfindungsgemäß einzusetzende siliziumorganische Beschichtungen gibt, bei denen die Adhäsion von gramnegativen und auch gleichzeitig von grampositiven Bakterien stark vermindert wird. Blutkompatibilitätstests zeigen, dass keine Koagulation auftritt.

Ein bei bestimmungsgemäßem Gebrauch mit Körperflüssigkeit eines Lebewesens oder einem ihrer Bestandteile umströmter oder durchströmter Gegenstand im Sinne der Erfindung ist vorzugsweise eine Membran, ein Rohr oder ein Schlauch, insbesondere eine Oxygenatormembran, ein Katheter, ein Angioplasieballon, ein Stent oder eine Kanüle.

Ein bei bestimmungsgemäßem Gebrauch implantierter Gegenstand im Sinne der Erfindung ist vorzugsweise ein Implantat, ein medizinischer Nagel, eine Klammer, ein Faden und/oder eine Schraube, insbesondere ein Knochenfixationsnagel, ein Stent, eine Kanüle, ein Sensor und eine Sonde oder eine Gefäßprothese, ein Injektionssystem, ein Katheter, ein kardiovaskuläres Implantat, ein künstliches Organ, insbesondere ein Herzschrittmacher und eine Stromquelle desselben, eine Prothese, ein orthopädisches Implantat, insbesondere ein künstlicher Gelenkkörper, insbesondere eine Gelenkpfanne und ein damit zusammenwirkendes Gegenstück, wie eine Hüft- oder Knieprothese, eine Wirbelsäulenprothese, ein Cochlea-Implantat, ein Dentalimplantat, eine künstliche Herzklappe, ein Herzklappenring, eine Intraocularlinse, eine künstliche Hornhaut, eine Pumpe oder eine andere Vorrichtung zur Freisetzung von Substanzen im Körper und Epithese.

Ein Behältnis zur Aufnahme und/oder zum Transport von Körperflüssigkeit, Gewebe oder deren Bestandteilen eines Lebewesens oder von Biomolekülen, bevorzugt Peptiden, Proteinen, Lipiden, Nukleinsäuren oder damit hergestellten Wirkstoffen im Sinne der Erfindung ist vorzugsweise eine Schale, ein Blutkonserven-Beutel, ein Zellkultur-Behältnis, ein Fermenter.

Ein Gegenstand zum zumindest teilweisen Bedecken von Haut oder Schleimhaut eines Lebewesens, und bevorzugt von Wunden, im Sinne der Erfindung ist vorzugsweise eine Wundauflage, ein Wundverband, ein Pflaster, eine Kontaktlinse, ein Inkontinenzprodukt.

Ein bei bestimmungsgemäßem Gebrauch anderweitig mit Körperflüssigkeit, Gewebe oder deren Bestandteilen eines Lebewesens oder mit Biomolekülen, bevorzugt Peptiden, Proteinen, Nukleinsäuren oder damit hergestellten Wirkstoffen in Kontakt stehender Gegenstand im Sinne der Erfindung ist vorzugsweise
- ein Sensor, insbesondere zur in-vivo-Analyse oder zur ex-vivo-Analyse, bevorzugt ein Sensor zur Untersuchung von Körperflüssigkeit, insbesondere Blut, Serum und Lymphe, Körperzellen, Gewebe, Organ(en),
- eine Sonde zum Einsatz im menschlichen oder tierischen Körper, bevorzugt eine Magensonde, ein Endoskop, eine Sonde zur Messung des Hirndrucks,
- eine Pumpe oder andere Vorrichtung zur Freisetzung von Substanzen im Körper
- ein Arzneimittelbehältnis, insbesondere enthaltend peptid-, protein-, fettsäure- und/oder nukleinsäurehaltige Inhalte, insbesondere Liposomen und/oder DNS-basierte Wirkstoffe oder Transportvehikel
- ein Teil eines Bioreaktors, auf dem Biokatalysatoren, wie zum Beispiel Enzyme oder Mikroorganismen, fixiert werden,
- ein Filtermaterial zum Herausfiltern von Zellen und/oder biologisch herstellbaren Makromolekülen aus einer Flüssigkeit.

Bevorzugt ist eine erfindungsgemäß zu verwendende Schicht auf dem Substrat wischbeständig. "Wischbeständig" im Sinne dieses Textes bedeutet beständig gegenüber einmaligem händischen Wischen mit Isopropanol-getränkten 2-lagigen Kosmetik- und Allzwecktüchern aus Soft-Tissue (beispielsweise der Firma TEMCA GmbH). Ebenfalls bevorzugt weisen die erfindungsgemäß zu verwendenden Schichten ein Elastizitätsmodul von mehr als 0,5 GPa oder eine Härte von mehr als 0,05 GPa auf, besonders bevorzugt ein Elastizitätsmodul von mehr als 0,8 GPa oder eine Härte von mehr als 0,07 GPa, jeweils gemessen mit Hilfe von Nanoindentierung. Das für die Messung im Zweifelsfall maßgebliche Messverfahren ist in der WO 2009/056635 A2, Beispiel 2 beschrieben, welches auf dem Wege der Verweisung Bestandteil dieses Textes wird.

Des Weiteren ist es bevorzugt, eine erfindungsgemäß zu verwendende Schicht oder einen erfindungsgemäßen Gegenstand (beim Beschichten) herzustellen in Gegenwart von sauerstoffhaltigen Gasen, die weder Kohlenstoff noch Silizium (auch jeweils in Verbindungen) enthalten. Solche Gase sind also dann zusätzlich zu eventuellen gasförmigen Precursoren oder Fragmenten der Precursoren bei der Erzeugung der Schicht zugegen. Bevorzugte Beispiele für solche Gase sind O₂, N₂O und H₂O.

Bevorzugt ist häufig einerseits ein erfindungsgemäßer Gegenstand, dessen nicht-genotoxische Oberfläche im Bereich der erfindungsgemäß einzusetzenden Schicht ein atomares Verhältnis von Sauerstoff zu Silizium von 1,6 bis 2,2, ein atomares Verhältnis von Kohlenstoff zu Silizium von 0,1 bis 0,5 und ein atomares Verhältnis von Kohlenstoff zu Sauerstoff von 0,01 bis 0,2 aufweist. Derartige Schichten sind insbesondere hämokompatibel und dementsprechend besonders geeignet als Beschichtung von Oxygenatormembranen und anderen mit Blut in Berührung kommenden Oberflächen, insbesondere von Implantaten. Derartige Schichten besitzen bevorzugt einen Wasser-Kontaktwinkel von nicht mehr als 35°, vorzugsweise nicht mehr als 25° und besonders bevorzugt von 0-15°.

Bevorzugt ist häufig andererseits auch ein erfindungsgemäßer Gegenstand, dessen nicht-genotoxische Oberfläche im Bereich der erfindungsgemäß einzusetzenden Schicht ein atomares Verhältnis von Sauerstoff zu Silizium von 0,75 bis 2,0, ein atomares Verhältnis von Kohlenstoff zu Silizium von 0,6 bis 2,5 und ein atomares Verhältnis von Kohlenstoff zu Sauerstoff von 0,4 bis 3,0 und ein atomares Verhältnis von Wasserstoff zu Kohlenstoff von 1,5 bis 3,2 aufweist.

An derartig beschichteten Gegenständen ist die Adhäsionsneigung biologischen Materials, insbesondere von Bakterien, Pilzen und eukaryontischen Zellen, stark herabgesetzt im Vergleich zu Metalloberflächen wie Titanoberflächen. Diese Oberflächen eignen sich daher besonders für solche erfindungsgemäßen Gegenstände, die frei von Bewuchs oder Anlagerung biologischen Materials bleiben sollen, wie Kanülen und Arzneimittelbehältnisse. Nachfolgend soll diese nicht-genotoxischen Schicht als "Biokompatibilitätsschicht A" bezeichnet werden.

Besonders bevorzugt gelten für die Biokompatibilitätsschicht A dieser Gegenstände die folgenden Stoffmengenverhältnisse:

| | | |
|---|---|---|
| 0,85 | < n(O) : n(Si) < | 1,8 |
| 0,8 | < n(C) : n(Si) < | 2,8 |
| 0,5 | < n(C) : n(O) < | 2,6 |
| 1,8 | < n(H) : n(C) < | 3,1. |

Ganz besonders bevorzugt gelten für die Biokompatibilitätsschicht A dieser Gegenstände:

| | | |
|---|---|---|
| 1,0 | < n(O) : n(Si) < | 17 |
| 1,4 | < n(C) : n(Si) < | 2,6 |
| 0,9 | < n(C) : n(O) < | 2,4 |
| 2,2 | < n(H) : n(C) < | 3,0. |

Hierbei beziehen sich die Stoffmengenverhältnisse aller Atompaarungen ohne Wasserstoff auf XPS-Messungen bei Einstellungen, welche für ein als Standard eingesetztes trimethylsiloxy-terminiertes Polydimethylsiloxan (PDMS) mit einer kinematischen Viskosität von 350 mm²/s bei 25 °C und einer Dichte von 0,97 g/mL bei 25 °C Stoffmengenverhältnisse von n(O) : n(Si) = 1,02, n(C) : n(Si) = 2,35 und n(C) : n(O) = 2,29 ergeben. Das Verhältnis zwischen Wasserstoff und Kohlenstoff bezieht sich auf Ergebnisse der klassischen chemischen Elementaranalyse.

Hinsichtlich der Stoffmengenanteile der Elemente Silizium, Sauerstoff und Kohlenstoff gilt bevorzugt, dass die Biokompatibilitätsschicht A dieser Gegenstände, bezogen auf 100 Atom-% für die Summe der Elemente Silizium, Sauerstoff und Kohlenstoff, enthält:

| | |
|---|---|
| Silizium: | 18 bis 30 Atom-% |
| Sauerstoff: | 20 bis 50 Atom-% |
| Kohlenstoff: | 25 bis 60 Atom-%. |

Besonders bevorzugt ist es aber, wenn die Biokompatibilitätsschicht A dieser Gegenstände, bezogen auf 100 Atom-% für die Summe der Elemente Silizium, Sauerstoff und Kohlenstoff, enthält:

| | |
|---|---|
| Silizium: | 20 bis 28 Atom-% |
| Sauerstoff: | 22 bis 45 Atom-% |
| Kohlenstoff: | 35 bis 55 Atom-%. |

Hierbei beziehen sich die Atom-%-Angaben auf XPS-Messungen bei Einstellungen, welche für ein wiederum als Standard eingesetztes trimethylsiloxy-terminiertes Polydimethylsiloxan (PDMS) mit einer kinematischen Viskosität von 350 mm²/s bei 25 °C und einer Dichte von 0,97 g/mL bei 25 °C für Silizium 22,9 Atom-%, für Sauerstoff 23,4 Atom-% und für Kohlenstoff 53,75 Atom-% ergeben.

Unter Berücksichtigung bevorzugter Gewichtsanteile und Stoffmengenverhältnisse ist die Biokompatibilitätsschicht A dieser Gegenstände besonders bevorzugt, wenn sie, bezogen auf 100 Atom-% für die Summe der Elemente Silizium, Sauerstoff und Kohlenstoff, enthält:

| | |
|---|---|
| Silizium: | 18 bis 30 Atom-% |
| Sauerstoff: | 20 bis 50 Atom-% |
| Kohlenstoff: | 25 bis 60 Atom-%, |

wobei für die Stoffmengenverhältnisse in der Biokompatibilitätsschicht A dieser Gegenstände gilt:

| | | |
|---|---|---|
| 0,75 | < n(O) : n(Si) < | 2,0 |
| 0,6 | < n(C) : n(Si) < | 3,0 |
| 0,4 | < n(C) : n(O) < | 3,0 |
| 1,5 | < n(H) : n(C) < | 3,2 und |

wobei im XPS-Spektrum der Biokompatibilitätsschicht dieser Gegenstände im Vergleich mit einem trimethylsiloxy-terminierten Polydimethylsiloxan (PDMS) mit einer kinematischen Viskosität von 350 mm²/s bei 25 °C und einer Dichte von 0,97 g/mL bei 25 °C,
der Si 2p Peak einen Bindungsenergiewert besitzt, der um maximal 0,8 eV zu höheren oder niedrigeren Bindungsenergien verschoben ist und
der O 1s Peak einen Bindungsenergiewert besitzt, der um maximal 0,7 eV zu höheren oder niedrigeren Bindungsenergien verschoben ist.

Hinsichtlich der XPS-Messbedingungen und dem gewählten Standard gilt hierbei das oben Gesagte entsprechend.

Eine ganz besonders bevorzugte Biokompatibilitätsschicht A dieser Gegenstände enthält, bezogen auf 100 Atom-% für die Summe der Elemente Silizium, Sauerstoff und Kohlenstoff:

| | |
|---|---|
| Silizium: | 20 bis 28 Atom-% |
| Sauerstoff: | 22 bis 32 Atom-% |
| Kohlenstoff: | 38 bis 53 Atom-%, |

wobei für die Stoffmengenverhältnisse der Biokompatibilitätsschicht A dieser Gegenstände gilt:

| | | |
|---|---|---|
| 1,0 | < n(O) : n(Si) < | 17 |
| 1,4 | < n(C) : n(Si) < | 2,6 |
| 0,9 | < n(C) : n(O) < | 2,4 |
| 2,2 | < n(H) : n(C) < | 3,0 und |

wobei im besagten ESCA-Spektrum der Si 2p Peak einen Bindungsenergiewert besitzt, der um maximal 0,60 eV zu höheren oder niedrigeren Bindungsenergien verschoben ist und der O 1s Peak einen Bindungsenergiewert besitzt, der um maximal 0,65 eV zu höheren oder niedrigeren Bindungsenergien verschoben ist.

Dabei können in bevorzugten Ausführungsformen die für eine erfindungsgemäße Verwendung zur Herabsetzung der Adhäsion ausgeschlossenen Schichten auch für die erfindungsgemäßen nicht-genotoxischen Gegenstände ausgeschlossen sein.

Ebenfalls bevorzugt ist eine Biokompatibilitätsschicht A, die einen Anteil von 0,1 bis ≤ 5% an Kohlenstoffatomen mit einer Bindung zu genau zwei Sauerstoff-Atomen ("COO") aufweist, gemessen mit XPS. Entsprechend liegen maximal 5% der Kohlenstoffatome der siliziumhaltigen Schicht als Ester- oder Säuregruppen vor. Stärker bevorzugt ist ein Anteil von ≤ 3%.

Zudem bevorzugt ist eine Biokompatibilitätsschicht A, die einen Anteil von ≤ 12% an Kohlenstoffatomen mit einer Bindung zu genau einem Sauerstoff-Atom ("C-O") aufweist, gemessen mit XPS. Entsprechend liegen maximal 12% der Kohlenstoffatome der siliziumhaltigen Schicht als Hydroxy- oder Ethergruppen vor. Stärker bevorzugt ist ein Anteil von ≤ 8%.

Hierdurch wird effektiv die Adhäsion von Biomolekülen gesenkt.

Der Anteil an Kohlenstoffatomen mit einer Bindung zu genau einem Sauerstoff-Atom ("C-O") sowie zu genau zwei Sauerstoff-Atomen ("COO") wird ermittelt, indem die XPS-Spektren, welche zur Probennormalen orientiert gemessen worden sind, einer Kurvenanpassung (einem Curve-Fitting) unterzogen werden. Unter der Vorraussetzung, dass die Stickstoffkonzentration in der Beschichtung klein ist (< 0.5 Atom-%) werden die chemischen Verschiebungen (Shifts) beim C1s-Spektrum wie in Tabelle 1 angegeben interpretiert:

| Abkürzung | Peaknummer (in den Fig. 3-8) | Gruppen | Bindungsenergie |
|---|---|---|---|
| "C" | I | Aliphatisch | 285,0 eV |
| "C-O" | II | Alkohol, Ether | 286.5 ± 0.2 eV |
| "COO" | III | Carboxyl, Ester | 289.2 ± 0.2 eV |

Für eine erfindungsgemäße Biokompatibilitätsschicht A kann es fallweise bevorzugt sein, dass der Kontaktwinkel auf einer ebenen Oberfläche bei 25°C einen Wasser-Kontaktwinkel von zumindest 90°, vorzugsweise zumindest 95° und besonders bevorzugt zumindest 100° darstellt.

Insbesondere bevorzugt ist ein erfindungsgemäßer Gegenstand mit einer nicht-genotoxischen Schicht, die zu mehr als 98% aus den Elementen Silizium, Kohlenstoff, Sauerstoff, Wasserstoff und Stickstoff besteht. Weiter bevorzugt ist ein erfindungsgemäßer Gegenstand mit einer nicht-genotoxischen Schicht, die zu mehr als 98% aus den Elementen Silizium, Kohlenstoff, Sauerstoff und Wasserstoff besteht. Solche Schichten lassen sich, wie nachfolgend noch weiter beschrieben wird, besonders gut zum Verwirklichen der oben genannten Vorteile herstellen.

Nicht-genotoxischen Schichten für Anwendungen, für die besonders hydrophile und hämokompatible Oberflächen gefordert sind, werden nachfolgend als Biokompatibilitätschichten B bezeichnet. In dieser speziellen Ausgestaltung der Erfindung weisen die Kohlenstoffatome der Biokompatibilitätsschicht B vorzugsweise einen Anteil von 5-35% an Kohlenstoffatomen mit einer Bindung zu genau einem Sauerstoff-Atom ("C-O") auf, gemessen mit XPS. Entsprechend liegen 5-35% der Kohlenstoffatome der siliziumhaltigen Schicht als Hydroxyl- oder Ethergruppen vor. Stärker bevorzugt ist ein Anteil von 10-30%, vorzugsweise von 15-25%. Auf diese Weise lassen sich besonders hydrophile und hämokompatible Schichten herstellen. Insbesondere haben diese bevorzugten Bindungsvarianten den Vorteil, dass die Adhäsion von Biomolekülen insbesondere bevorzugt Albumin und Fibromectin verbessert wird, wodurch wiederum das Zellwachstum verbessert wird.

Besonders bevorzugt gelten für die Biokompatibilitätsschicht B dieser Gegenstände die folgenden Stoffmengenverhältnisse:

| | | |
|---|---|---|
| 1,8 | < n(O) : n(Si) < | 3,0 |
| 0,1 | < n(C) : n(Si) < | 1,2 |
| 0,05 | < n(C) : n(O) < | 0,6 |
| 0,5 | < n(H) : n(C) < | 3,0. |

Ganz besonders bevorzugt gelten für die Biokompatibilitätsschicht B dieser Gegenstände:

| | | |
|---|---|---|
| 2,2 | < n(O) : n(Si) < | 2,5 |
| 0,2 | < n(C) : n(Si) < | 0,7 |
| 0,05 | < n(C) : n(O) < | 0,4 |
| 1,0 | < n(H) : n(C) < | 2,2. |

Hierbei beziehen sich die Stoffmengenverhältnisse aller Atompaarungen ohne Wasserstoff auf XPS-Messungen bei Einstellungen, welche für ein als Standard eingesetztes trimethylsiloxy-terminiertes Polydimethylsiloxan (PDMS) mit einer kinematischen Viskosität von 350 mm²/s bei 25 °C und einer Dichte von 0,97 g/mL bei 25 °C Stoffmengenverhältnisse von n(O) : n(Si) = 1,02, n(C) : n(Si) = 2,35 und n(C) : n(O) = 2,29 ergeben. Das Verhältnis zwischen Wasserstoff und Kohlenstoff bezieht sich auf Ergebnisse der klassischen chemischen Elementaranalyse.

Hinsichtlich der Stoffmengenanteile der Elemente Silizium, Sauerstoff und Kohlenstoff gilt bevorzugt, dass die Biokompatibilitätsschicht B dieser Gegenstände, bezogen auf 100 Atom-% für die Summe der Elemente Silizium, Sauerstoff und Kohlenstoff, enthält:

| | |
|---|---|
| Silizium: | 18 bis 32 Atom-% |
| Sauerstoff: | 45 bis 70 Atom-% |
| Kohlenstoff: | 3 bis 25 Atom-%. |

Besonders bevorzugt ist es aber, wenn die Biokompatibilitätsschicht B dieser Gegenstände, bezogen auf 100 Atom-% für die Summe der Elemente Silizium, Sauerstoff und Kohlenstoff, enthält:

| | |
|---|---|
| Silizium: | 24 bis 30 Atom-% |
| Sauerstoff: | 50 bis 68 Atom-% |
| Kohlenstoff: | 3 bis 19 Atom-%. |

Hierbei beziehen sich die Atom-%-Angaben auf XPS-Messungen bei Einstellungen, welche für ein wiederum als Standard eingesetztes trimethylsiloxy-terminiertes Polydimethylsiloxan (PDMS) mit einer kinematischen Viskosität von 350 mm²/s bei 25 °C und einer Dichte von 0,97 g/mL bei 25 °C für Silizium 22,9 Atom-%, für Sauerstoff 23,4 Atom-% und für Kohlenstoff 53,75 Atom-% ergeben.

Unter Berücksichtigung bevorzugter Gewichtsanteile und Stoffmengenverhältnisse ist die Biokompatibilitätsschicht B dieser Gegenstände besonders bevorzugt, wenn sie, bezogen auf 100 Atom-% für die Summe der Elemente Silizium, Sauerstoff und Kohlenstoff, enthält:

| | |
|---|---|
| Silizium: | 18 bis 32 Atom-% |
| Sauerstoff: | 45 bis 70 Atom-% |
| Kohlenstoff: | 3 bis 25 Atom-% und |

wobei für die Stoffmengenverhältnisse in der Biokompatibilitätsschicht B dieser Gegenstände gilt:

| | | |
|---|---|---|
| 1,8 | < n(O) : n(Si) < | 3,0 |
| 0,1 | < n(C) : n(Si) < | 1,2 |
| 0,05 | < n(C) : n(O) < | 0,6 |
| 0,5 | < n(H) : n(C) < | 3,0 |

Hinsichtlich der XPS-Messbedingungen und dem gewählten Standard gilt hierbei das oben Gesagte entsprechend.

Eine ganz besonders bevorzugte Biokompatibilitätsschicht B dieser Gegenstände enthält, bezogen auf 100 Atom-% für die Summe der Elemente Silizium, Sauerstoff und Kohlenstoff:

| | |
|---|---|
| Silizium: | 24 bis 30 Atom-% |
| Sauerstoff: | 50 bis 68 Atom-% |
| Kohlenstoff: | 3 bis 19 Atom-%. |

wobei für die Stoffmengenverhältnisse der Biokompatibilitätsschicht B dieser Gegenstände gilt:

| | | |
|---|---|---|
| 2,2 | < n(O) : n(Si) < | 2,5 |
| 0,2 | < n(C) : n(Si) < | 0,7 |
| 0,05 | < n(C) : n(O) < | 0,4 |
| 1,0 | < n(H) : n(C) < | 2,2. |

Zudem weist die erfindungsgemäße Biokompatibilitätsschicht B dieser Gegenstände beim Messen mit fortschreitendem Kontaktwinkel auf einer ebenen Oberfläche bei 25°C einen Wasser-Kontaktwinkel von weniger als 70°, vorzugsweise zumindest weniger als 60° und besonders bevorzugt weniger als 50° auf.

Bevorzugt ist die erfindungsgemäße Biokompatibilitätsschicht B beständig gegenüber dem händischen Wischen mit Isopropanol-getränkten 2-lagigen Kosmetik- und Allzwecktüchern, aus Soft-Tissue (beispielsweise der Firma TEMCA GmbH). Ebenfalls bevorzugt weisen die erfindungsgemäß zu verwendenden Schichten ein Elastizitätsmodul von mehr als 3,0 GPa oder eine Härte von mehr als 0,4 GPa auf, besonders bevorzugt ein Elastizitätsmodul von mehr als 5,0 GPa oder eine Härte von mehr als 0,5 GPa, jeweils gemessen mit Hilfe von Nanoindentierung, wobei die oben beschriebene Messmethode eingesetzt wird.

Ebenfalls bevorzugt ist eine Biokompatibilitätsschicht B, die einen Anteil von 5-20% an Kohlenstoffatomen mit einer Bindung zu genau zwei Sauerstoff-Atomen ("COO") aufweist, gemessen mit XPS. Entsprechend liegen 5-20% der Kohlenstoffatome der siliziumhaltigen Schicht als Ester- oder Säuregruppen vor. Stärker bevorzugt ist ein Anteil von 5-10%. Diese polaren Gruppen fördern ebenfalls die Hydrophilie der erfindungsgemäßen Biokompatibilitätsschicht. Insbesondere bevorzugen auch diese Ausgestaltungen die Adhäsion von Biomolekülen wie ganz besonders Albumin und Fibromectin, was wiederum das Zellwachstum verbessert.

Der Anteil an Kohlenstoffatomen mit einer Bindung zu genau einem Sauerstoff-Atom ("C-O") sowie zu genau zwei Sauerstoff-Atomen ("COO") wird ermittelt, indem die XPS-Spektren, welche zur Probennormalen orientiert gemessen worden sind, einer Kurvenanpassung (einem Curve-Fitting) unterzogen werden. Unter der Vorraussetzung, dass die Stickstoffkonzentration in der Beschichtung klein ist (< 0.5 Atom-%) werden die chemischen Verschiebungen (Shifts) beim C1s-Spektrum wie in Tabelle 1 angegeben interpretiert:

**Tabelle 1**

| Abkürzung | Peaknummer (in den Fig. 3-8) | Gruppen | Bindungsenergie |
|---|---|---|---|
| "C" | I | Aliphatisch | 285,0 eV |
| "C-O" | II | Alkohol, Ether | 286.5 ± 0.2 eV |
| "COO" | III | Carboxyl, Ester | 289.2 ± 0.2 eV |

Der aliphatische Kohlenstoff wird per Definition auf 285,0 eV gesetzt. Zur Vorbereitung des Curve-Fittings wird eine Baseline-Korrektur des C1s-Spektrums nach Shirley im Bereich von 281 eV bis 292 eV durchgeführt. Anschließend wird mit Hilfe von Gauß-Lorentz-Funktionen gefittet. Hierzu wird das Maximum der Bindungsenergie für den aliphatischen Kohlenstoff auf 285.0 eV, das Maximum des "C-O"-Kohlenstoffs auf 286.5 eV bzw. auf 289.2 eV für den "COO"-Kohlenstoff gelegt. Als Fitparameter wird die Energie im Bereich von ± 0.2 eV, die Zählrate und die Halbwertsbreite angesetzt. Dabei wird die Halbwertsbreite auf maximal 1,4 eV festgesetzt. Die Bindungsenergie des aliphatischen Kohlenstoffs wird nicht mit angefittet. Der Fit ist dann beendet, wenn der Least-Square-Fit-Algorithmus sein Minimum annimmt. Aus den Flächenverhältnissen der Funktion für den aliphatischen Kohlenstoff bzw. den Funktionen für "C-O"- und "COO"-Kohlenstoff lassen sich die gruppenspezifischen Konzentrationen berechnen. Weiterhin wird bei der Quantifizierung der Konzentrationen angenommen, dass sich die funktionellen Gruppen in der gesamten Informationstiefe des XPS-Spektrums homogen verteilen, so dass die gemessene Beschichtung mindestens 10 nm dick sein muss.

Die nicht-genotoxischen Schicht ist hergestellt oder herstellbar durch Plasmapolymerisation eines Methylsiloxan-Precursors, vorzugsweise von Hexamethyldisiloxan, insbesondere durch Niederdruck- oder Atmosphärendruck-Plasmapolymerisation, oder durch Vernetzen eines Silikonöls ohne chemisch reaktive Gruppen unter Einwirkung eines Plasmas oder UV-Strahlung einer Wellenlänge unter 250 nm, insbesondere Excimer-Strahlung.

Eine "plasmapolymere Schicht" ist im Rahmen dieses Textes eine Schicht, welche mittels Plasmapolymerisation herstellbar ist. Plasmapolymerisation ist ein Verfahren, bei dem sich gasförmige Precursoren (oft auch Monomere genannt), angeregt durch ein Plasma, auf einem frei wählbaren Substrat als hochvernetzte Schicht niederschlagen. Voraussetzung für eine Plasmapolymerisation ist das Vorhandensein von kettenbildenden Atomen wie Kohlenstoff oder Silizium im Arbeitsgas. Durch die Anregung werden die Moleküle der gasförmigen Substanz (Precursoren), durch den Beschuss mit Elektronen und/oder energiereichen Ionen fragmentiert. Dabei entstehen hochangeregte radikalische oder ionische Molekülfragmente, die miteinander im Gasraum reagieren und auf der zu beschichtenden Oberfläche abgeschieden werden. Auf diese abgeschiedene Schicht wirkt die elektrische Entladung des Plasmas und dessen intensiver Ionen- und Elektronenbeschuss fortwährend ein, so dass in der abgeschiedenen Schicht weitere Reaktionen ausgelöst und eine hochgradige Verknüpfung (Vernetzung) der abgeschiedenen Moleküle erzielt werden kann.

Auch Kombinationen der Beschichtungsverfahren sind möglich, beispielsweise die Plasmavernetzung von Siliconöl kombiniert mit einer Plasmabeschichtung.

Als Flüssigkeitsfilm wird im letzteren Fall auf die Oberfläche (z. B. des Trägers) zunächst eine Substanz aufgebracht, die kettenbildende Atome wie Kohlenstoff oder Silizium beinhaltet, z.B. Silikonöl. Wird der Flüssigkeitsfilm einem Plasma ausgesetzt, so wirken die Elektronen und/oder energiereichen Ionen und die im Plasma erzeugte UV-Strahlung auf die Flüssigkeitsmoleküle ein. In der Flüssigkeit entstehen Bindungsbrüche, die zu einer Vernetzung der Flüssigkeitsmoleküle führen. Über die Expositionsdosis kann der Fachmann einen geeigneten Vernetzungsgrad des Flüssigkeitsfilms erzielen.

Im Rahmen des vorliegenden Textes umfasst der Begriff "plasmapolymere Schicht" auch Schichten, die mittels plasmaunterstützter CVD (PE-CVD) hergestellt werden können.

Ferner sei ausdrücklich erwähnt, dass auch Atmosphärendruckplasmaverfahren zur Herstellung erfindungsgemäß einzusetzender plasmapolymerer Schichten verwendet werden können, wenngleich Niederdruck-Plasmapolymerisationsverfahren derzeit bevorzugt sind.

Im Rahmen des vorliegenden Textes werden Substanzen, die zur Schichtbildung über eine Plasmapolymerisation als Gas bzw. Dampf einem Plasma zugeführt werden, als "Monomere" (gasförmige Precursoren) bezeichnet. Als "flüssige Precursoren" werden Flüssigkeiten bezeichnet, welche beispielsweise durch die Einwirkung eines Plasmas vernetzt werden können (beispielsweise durch hochangeregte Teilchen, Elektronen oder UV-Strahlung), ohne vorher zu verdampfen.

Plasmapolymere Schichten sind in ihrer chemischen und strukturellen Zusammensetzung eindeutig von polymeren Schichten zu unterscheiden. Während bei Polymeren der Verknüpfungsprozess der Monomere in vorhersagbarer Weise geschieht, werden bei der Plasmapolymerisation die eingesetzten Monomere fragmentiert (bis zur vollständigen Zerstörung) und in Form von reaktiven Spezies abgeschieden, so dass sich eine vernetzte Schicht ergibt, ohne Bereiche mit regelmäßigen Wiederholungseinheiten. Diese entstehende Schicht ist zusätzlich noch dem Plasma ausgesetzt, so dass sie durch Ablation und Redepositionseffekte weiter modifiziert wird. Die entstehende Schicht ist dreidimensional vernetzt und amorph. Dementsprechend unterscheidet sich die Plasmapolymerisation im Sinne dieses Textes von konventionellen Methoden der Polymerisation. Dies gilt auch für die sogenannte "strukturerhaltende Plasmapolymerisation", da selbst bei "milden" Plasmabedingungen unvorhersagbare Molekülbrüche auftreten. In diesem Zusammenhang sei beispielsweise auf folgende Literaturstelle verwiesen: "Plasma Polymerization" by H. Yasuda, Academic Press, Inc., (1985).

Nach der Herstellung einer erfindungsgemäß einzusetzenden nicht-genotoxischen Schicht durch das Vernetzen des Precursors bzw. Silikonöls kann diese zumindest teilweise oxidiert werden, vorzugsweise durch Plasma-Einwirkung ("Aktivierung"), Beflammung, Oxy-Fluorierung, Laser-Behandlung oder Behandlung mit Excimer-Lampen. Insbesondere durch Oxidation kann die chemische Zusammensetzung der Oberfläche der Beschichtung auf einfache Weise definiert eingestellt werden. Auf diese Weise ist es beispielsweise möglich, die oben aufgezählten verschiedenen erfindungsgemäßen Gegenstände mit einer Biokompatibilitätsschicht B zu versehen, insbesondere mit einer Schicht, auf der sich direkt nach der Herstellung ein Wasserkontaktwinkel von nicht mehr als 35° einstellt, um die oben aufgezählten Vorteile zu erreichen. Insbesondere lassen sich auf diesem Wege überraschend effektiv die oben beschriebenen speziellen Stoffverhältnisse (insbesondere C-O/COO-Bindungsanteile) einstellen, die eine Adhäsion von Biomolekülen fördern.

Durch das Einstellen der chemischen Oberflächenzusammensetzung allgemein ist insbesondere möglich,
- Fördern eines Anhaftens von Gewebezellen, bevorzugt mit einer Biokompatibilitätsschicht B, und/oder
- Reduzieren einer Anhaftung von Gewebezellen, bevorzugt mit einer Biokompatibilitätsschicht A, und/oder
- Reduzieren einer Anhaftung von Gewebezellen auf einem Teil der Oberfläche des biokompatiblen Gegenstands, bevorzugt mit einer Biokompatibilitätsschicht A, und Fördern der Anhaftung auf einem anderen Teil der Oberfläche des nicht-genotoxischen Gegenstands, bevorzugt mit einer Biokompatibilitätsschicht B, und/oder
- Reduzieren einer Anhaftung pathogener Ablagerungen, bevorzugt mit einer Biokompatibilitätsschicht A, und/oder
- Reduzieren einer Anhaftung von Bakterien und/oder
- Reduzieren der Thrombogenität und/oder
- Reduzieren des Auftretens humoraler und zellulärer Immunreaktionen und/oder
- Reduzieren von unspezifischer Adsorption von Peptiden, Proteinen, Lipiden und/oder Nukleinsäuren an der Oberfläche des nicht-genotoxischen Gegenstands, bevorzugt mit einer Biokompatibilitätsschicht A, und/oder
- Ermöglichen räumlich begrenzten Wachstums von Zellkulturen auf der Oberfläche des biokompatiblen Gegenstands, bevorzugt durch nur teilweises Versehen der Oberfläche mit einer erfindungsgemäß einzusetzenden nicht-genotoxischen Schicht, bevorzugt mit einer Biokompatibilitätsschicht B, und/oder,
- Erhöhen der Benetzbarkeit durch wässrige Flüssigkeiten, insbesondere Blut, bevorzugt mit einer Biokompatibilitätsschicht B.

Zum Begrenzen des zu oxidierenden Bereichs der Nicht-genotoxischen Schicht wird vorzugsweise eine stoffschlüssig aufliegende Maske, vorzugsweise eine ablösbare selbstklebende Maske, besonders bevorzugt ein Klebeband, und/oder ein, vorzugsweise in Wasser, zumindest teilweise löslicher Stoff oder eine gedruckte Maske verwendet und vorzugsweise nach dem Oxidieren entfernt. Auf diese Weise ist eine besonders einfache Begrenzung des oxidierten Bereiches möglich.

Durch einfache Maskierungen oder z.B. lokale Laserbehandlung können auch sowohl wachstumsfördernde als auch wachstumshemmende Oberflächenbereiche auf ein und demselben Bauteil realisiert werden. Hierfür wird beispielsweise zur Wachstumshemmung die Biokompatibilitätsschicht A aufgebracht und zur Wachstumsförderung die Biokompatibilitätsschicht B aufgebracht oder die Biokompatibilitätsschicht A durch Oxidation in die Biokompatibilitätsschicht B umgewandelt.

Der Vorteil in einer teilweisen Beschichtung oder von unterschiedlichen Beschichtungen (wiederum bevorzugt durch das Vorsehen entsprechender Maskierungen erzeugt) liegt insbesondere darin, dass hier sehr einfach lokal unterschiedliche Eigenschaften (verbesserte/herabgesetzte Zelladhäsion) an den entsprechenden Stellen des erfindungsgemäßen Gegenstandes realisiert werden können. Dabei ist das zunächst vollständige Beschichten des Gegenstandes mit einer erfindungsgemäß nicht-genotoxischen Schicht wie z.B. der Biokompatibilitätsschicht A besonders ökonomisch, wenn anschließend ausgewählte Bereiche durch Oxidation in eine erfindungsgemäße nicht-genotoxische Schicht, wie z.B. die Biokompatibilitätsschicht B umgewandelt werden. Auf diese Art werden bei nur einmaligem Einsatz von entsprechenden Masken bzw. Beschichtungsbegrenzungen unterschiedliche Oberflächeneigenschaften bei Aufrechterhaltung der Nicht-Genotoxizität erzeugt.

Die nicht-genotoxischen Schicht besitzt vorzugsweise eine Schichtdicke von nicht mehr als 2 µm, bevorzugt nicht mehr als 1 µm, ferner bevorzugt nicht mehr als 500 nm, und dabei jeweils von zumindest 5 nm, vorzugsweise zumindest 10 nm und besonders bevorzugt von zumindest 15 nm.

Erfindungsgemäß wird insbesondere auch ein nicht-genotoxisch beschichteter Gegenstand angegeben, umfassend eine nicht-genotoxische Schicht wie zuvor beschrieben. Die Schicht ist dabei vorzugsweise angeordnet auf einer Außen- und/oder Innen-Oberfläche des beschriebenen erfindungsgemäßen Gegenstandes. Zweckmäßigerweise wird die Schicht auf derjenigen Oberfläche angeordnet, die bei bestimmungsgemäßem Gebrauch des Gegenstandes mit dem zu schützenden Gut in Kontakt kommt, also beispielsweise die Innenseite einer Spritze bzw. die Außenseite eines Knochenfixationsnagels.

Ein Teil der Erfindung ist ferner die Verwendung einer Nicht-genotoxischen Schicht zum Beeinflussen, Modifizieren oder Ändern der Zytotoxizität, gemessen gemäß ISO 10993-5:2003, der Reizungen oder intrakutaner Reaktivität, gemessen gemäß ISO 10993-10:2003, der systemischen Toxizität (akute Toxizität, subakute Toxizität und subchronische Toxizität), gemessen gemäß ISO 10993-11:2003, der Implantationsverträglichkeit, gemessen gemäß ISO 10993-6:2003, und/oder der Hämokompatibilität, gemessen gemäß ISO 10993-4:2003 eines Gegenstandes.

Aufgabe der vorliegenden Erfindung war es, Mittel anzugeben, mit denen die Übertragung von Funktionsschichten auf gegebenenfalls entstehende Substratoberflächen sauber, mit einer guten Beschichtungsqualität und in bereits bekannte Arbeitsprozesse gut integrierbar bewerkstelligt werden kann.

Bevorzugt wird die Biokompatibilitätsschicht A verwendet für folgende Anwendungen:
- Angioplasieballons
- Kanülen
- Blutkonserven-Beutel
- Sensoren
- Behältnisse zur Aufnahme und/oder zum Transport von Körperflüssigkeit, Gewebe oder deren Bestandteilen eines Lebewesens oder von Biomolekülen oder damit hergestellten Wirkstoffen
- Katheter wie Blasenkatheter, Koronarkatheter oder Insulinkatheter auf den Oberflächen, die nicht Einwachsen sollen
- Blutgefäßstents im Innenbereich
- künstliches Organe wie künstliche Nieren oder Herzen auf den Oberflächen, auf denen kein Einwachsen erwünscht ist
- Herzschrittmacher und eine Stromquelle desselben auf den Oberflächen, auf denen kein Einwachsen erwünscht ist wie beispielsweise Oberflächen, die zur Erneuerung der Stromversorgung freigelegt werden müssen.
- künstliche Herzklappen auf den Oberflächen, auf denen kein Einwachsen erwünscht ist, wie auf der Klappenoberfläche
- Zellkultur-Behältnisse
- Künstliche Hornhaut (Cornea) auf den Oberflächen, auf denen das Ansiedeln von Zellen verhindert werden muss, wie in dem für die Durchsicht verantwortlichen mittleren Bereich
- Fermenter, bei denen keine Wechselwirkungen mit der Reaktoroberfläche erwünscht sind
- Pumpen oder andere Vorrichtung zur Freisetzung von Substanzen im Körper auf den Oberflächen, die nicht von Zellen bedeckt werden sollen.

Bevorzugt wird die Biokompatibilitätsschicht B verwendet für folgende Anwendungen:
- Implantate (Endoprothesen) wie Knochenfixationsnägel, medizinische Nägel, Klammern, Fäden, Schrauben
- Cochlea -Implantate
- Herzklappenringe
- Intraocularlinsen
- Fermenter bei denen eine Wechselwirkung mit der Wandoberfläche erwünscht ist wie beispielsweise im Sinne einer heterogenen oder heterogenisierten Katalyse, besonders bevorzugt, wenn für die Reaktionen Zellen oder biologisch herstellbare Makromoleküle an Reaktoroberflächen fixiert werden
- Sonden wie perkutane endoskopische Jejunostomie-Sonden
- Filtermaterial zum Herausfiltern von Zellen und/oder biologisch herstellbaren Makromolekülen aus einem Fluid
- Biokompatible Trägermatrices (engl. scaffolds)
- Gegenstände zum zumindest teilweisen Bedecken von Haut oder Schleimhaut oder Wunden eines Lebewesens wie Wundverbände, Pflaster, Kontaktlinsen, Inkontinenzprodukte
- Pumpen oder andere Vorrichtung zur Freisetzung von Substanzen im Körper auf den Oberflächen, die Einwachsen sollen
- Prothesen (offene Implantate, Epithesen) im Bereich des Durchtritts durch die Haut bzw. das Gewebe
- Ports im Bereich des Durchtritts durch die Haut bzw. das Gewebe
- künstliche Gelenkkörper wie Gelenkpfannen und ein damit zusammenwirkende Gegenstücke beispielsweise für Hüftgelenke
- Wundauflagen
- Katheter wie Blasenkatheter, Koronarkatheter, Insulinkatheter in Bereichen, in denen ein Einwachsen erwünscht ist, besonders für Langzeitkatheter
- Blutgefäßstents auf den zur Gefäßwand gerichteten Oberflächen
- Injektionssysteme auf Oberflächen, auf denen ein Einwachsen erwünscht ist, besonders in Bereichen des Durchtritts durch die Haut bzw. das Gewebe
- künstliche Organe wie künstliche Nieren oder Herzen, besonders auf Kunststoffoberflächen, für die ein Einwachsen erwünscht ist
- Herzschrittmacher und eine Stromquelle desselben auf Bereichen für die ein Einwachsen erwünscht ist wie Elektroden, Kabel und Gehäuse - letzteres besonders auf den Oberflächen, die nicht beim Auswechseln der Stromquelle bewegt werden
- Zellkultur-Behältnisse bei denen ein Anwachsen von Zellen auf dem Behältnis erwünscht ist
- Künstliche Hornhäute (Cornea) im Randbereich, der einwachsen soll

Die Erfindung wird nachfolgend anhand der Beispiele und der Figuren näher beschrieben, ohne dass dadurch der Schutzbereich der Ansprüche eingeschränkt werden soll.

Die beigefügten Figuren 1 bis 16 zeigen:
- Figur 1:: XPS-Übersichtsspektrum der Beschichtung Plasmapolymer A1
- Figur 2:: XPS-Detailspektrum des O 1s Peaks der Beschichtung Plasmapolymer A1
- Figur 3:: XPS-Detailspektrum des C 1s Peaks der Beschichtung Plasmapolymer A1
- Figur 4:: XPS-Detailspektrum des Si 2p Peaks der Beschichtung Plasmapolymer A1
- Figur 5:: C1s-Spektrum der Beschichtung Plasmapolymer B4
- Figur 6:: C1s-Spektrum der Beschichtung Plasmapolymer B5
- Figur 7:: C1s-Spektrum der Beschichtung Plasmapolymer B6
- Figur 8:: C1s-Spektrum der Beschichtung Plasmapolymer B7
- Figur 9:: Zellwachstumsmanagement durch Biokompatibilitätsschichten auf Aluminium
- Figur 10:: Zellwachstumsmanagement durch Biokompatibilitätsschichten auf Edelstahl
- Figur 11:: Zellwachstumsmanagement durch Biokompatibilitätsschichten auf Glas
- Figur 12:: Zellwachstumsmanagement durch Biokompatibilitätsschichten unter Zuhilfenahme von Excimer-Lampen auf Glas
- Figur 13:: Zellwachstumsmanagement durch Biokompatibilitätsschichten auf Keramik
- Figur 14:: Zellwachstumsmanagement durch Biokompatibilitätsschichten auf PMMA
- Figur 15:: Zellwachstumsmanagement durch Biokompatibilitätsschichten auf Silikon
- Figur 16:: Zellwachstumsmanagement durch Biokompatibilitätsschichten auf Titan
- Figur 17:: Test auf Gentoxizität in Anlehnung an DIN EN ISO 10993-3 mit Hilfe des Kometen-Testverfahrens. Bilder zeigen links Negativkontrolle ohne DNS-Fragmentierung, Mitte Positivkontrolle mit ausgeprägtem Schweif und rechts Biokompatibilitätsschicht U1 ohne DNS-Fragmentierung.

### Beispiele

### Beispiel 1: Plasmapolymerbeschichtungen aus einem 1 m³ Reaktor

Flache Substrate der nachfolgend angeführten Werkstoffe wurden mittels eines Niederdruck-Plasmaverfahrens in einem 1m³ - Reaktor (Beschreibung siehe ISBN 978-3-86727-548-4 "Aufskalierung plasmapolymerer Beschichtungsverfahren", Seite 21 - 26 von Dr. Klaus Vissing) mit einer erfindungsgemäßen Schicht versehen:
- Aluminium als Abschnitte (ca. 10 x 10 mm)
- Edelstahl als Abschnitte (ca. 10 x 10 mm)
- Glas als Abschnitte (ca. 10 x 10 mm)
- Aluminiumoxid-Keramik Al23 (von Degussit- Friatec)
- Polymethylmethacrylat (PMMA) als Abschnitte (ca. 10 x 10 mm) aus Sterilverpackungsflaschen
- Silikon als Abschnitte (ca. 15*10 mm)
- Titan als Abschnitte (ca. 15*10 mm)

Hierfür wurden die Proben nach dem Stand der Technik im Plasma mit Sauerstoff feingereinigt sowie je nach Bedarf aktiviert und/oder mit einer Haftvermittlungsschicht versehen. Anschließend wurde bei einer Frequenz von 13,56 MHz die erfindungsgemäße plasmapolymere Beschichtung appliziert, wobei dem Plasma O₂ und Hexamethyldisiloxan (HMDSO) zugeführt wurden. Die genauen Verfahrensparameter für die Abscheidung der plasmapolymeren Beschichtung sind in Tabelle 2 angegebenen. Die Zeitspanne für die plasmapolymere Beschichtung variierte von 9 bis 60 Minuten. Hierbei wurden Schichten zwischen 90 und 250 nm Dicke aufgebracht. Die Abpumpzeit betrug bei Plasmapolymer A6 8,5 Minuten.

**Tabelle 2**

| **Beschichtung** | **Gasfluss HMDSO (Sccm)** | **Gasfluss O₂ (Sccm)** | **Gasfluss H₂ (Sccm)** | **Druck (mbar)** | **Leistung (W)** |
|---|---|---|---|---|---|
| Plasmapolymer A1 | 67 | 20 | | 0,025 | 700 |
| Plasmapolymer A2 | 27 | 20 | 200 | 0,025 | 1600 |
| Plasmapolymer A3 | 27 | 100 | | 0,023 | 2500 |
| Plasmapolymer A4 | 60 | 200 | | 0,020 | 700 |
| Plasmapolymer A5 | 30 | 200 | | 0,020 | 700 |
| Plasmapolymer A6 | 30 | 200 | | 0,024 | 1600 |

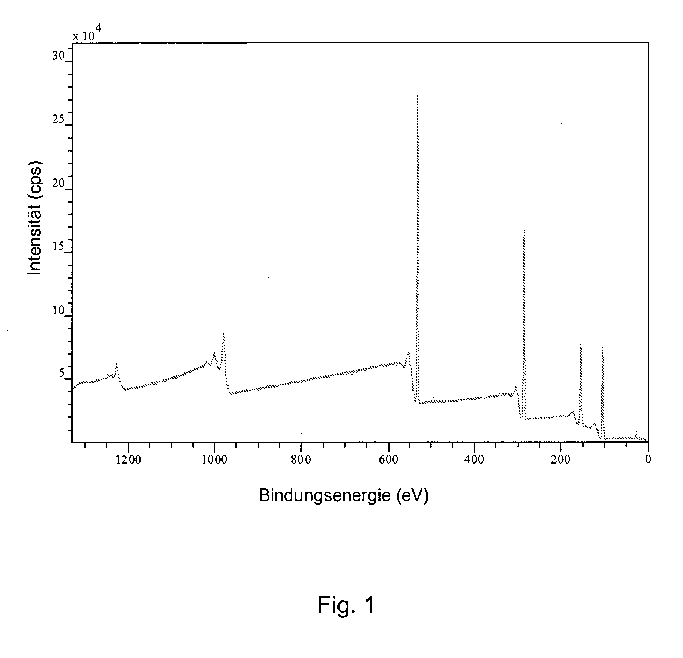

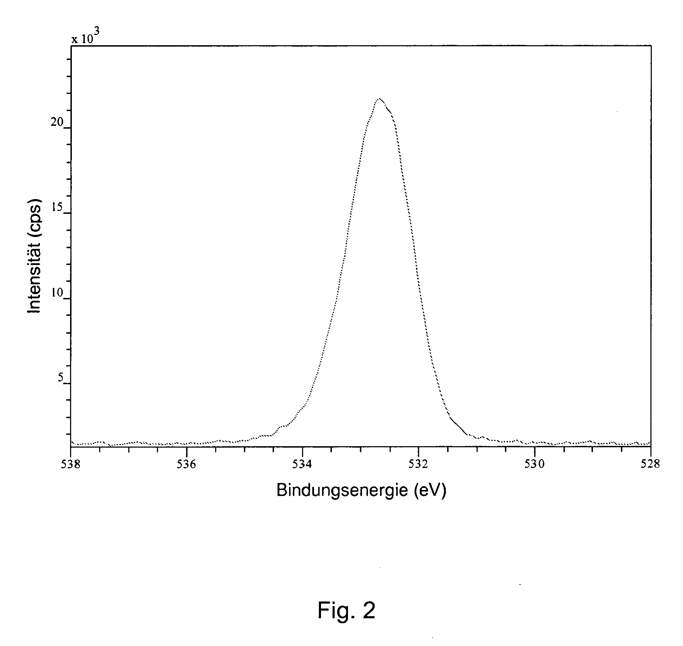

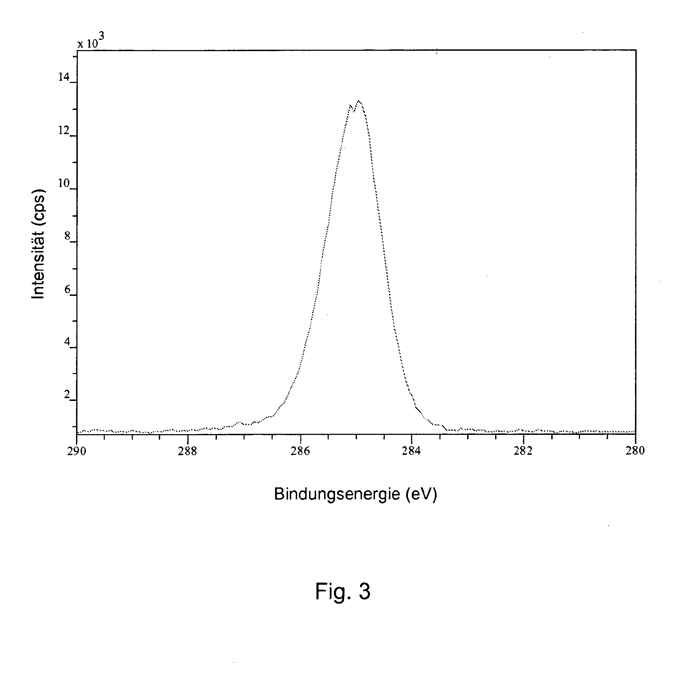

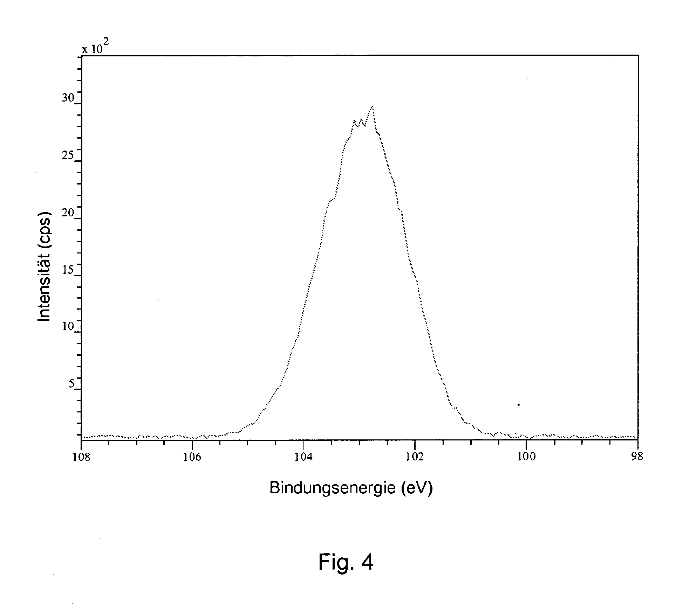

In den Figuren 1 bis 4 sind XPS-Spektren der Beschichtung Plasmapolymer A1 dargestellt.

Zudem wurden analog zum beschriebenen Verfahren für die Plasmapolymere A1 bis A3 plasmapolymere Beschichtungen unter Bedingungen wie in Tabelle 3 beschrieben hergestellt und dabei in einem weiteren Prozessschritt 60 Sekunden im Niederdruck-Plasma aktiviert. Für diese Schichten, die eine verbesserte Anhaftung von Biomolekülen und Zellen ermöglichten, sind die Prozessparameter für die plasmapolymere Beschichtung und die Aktivierung in Tabelle 3 angegeben. Die Zeitspanne für die plasmapolymere Beschichtung variierte von 12 bis 19 Minuten. Hierbei wurden Schichten zwischen 50 und 170 nm aufgebracht.

**Tabelle 3**

| | **Beschichtungsprozessschritt** | | | | **Aktivierungsprozessschritt** | | |
|---|---|---|---|---|---|---|---|
| | **Gasfluss** | | **Druck (mbar)** | **Leistung (W)** | **Gasfluss O₂ (sccm)** | **Druck (mbar)** | **Leistung (W)** |
| **Beschichtung** | **HMDSO (Sccm)** | **O₂ (Sccm)** | | | | | |
| Plasmapolymer B1 | 13 | 500 | 0,025 | 2000 | 500 | 0,025 | 2000 |
| Plasmapolymer B2 | 10 | 500 | 0,03 | 2000 | 500 | 0,03 | 2000 |
| Plasmapolymer B3 | 10 | 500 | 0,03 | 2000 | 500 | 0,025 | 2000 |

Daneben wurden ebenfalls analog zum beschriebenen Verfahren für die Plasmapolymere A1 bis A3 plasmapolymere Beschichtungen unter Bedingungen wie in Tabelle 4 beschrieben hergestellt und dabei in einem weiteren Prozessschritt kurzzeitig im Niederdruck-Plasma aktiviert. Für die entstandenen Schichten, die eine mittelmäßige bis gute Anhaftung von Biomolekülen und Zellen ermöglichten, sind die Prozessparameter für die plasmapolymere Beschichtung und die Aktivierung in Tabelle 4 angegeben. Die Beschichtungszeit für die plasmapolymere Beschichtung betrug 35 Minuten. Hierbei wurden Schichten einer Stärke von 104 und 110 nm aufgebracht.

**Tabelle 4**

| | **Beschichtungsprozessschritt** | | | | **Aktivierungsprozessschritt** | | | |
|---|---|---|---|---|---|---|---|---|
| | **Gasfluss** | | **Druck (mbar)** | **Leistung (W)** | **Gasfluss O₂ (Sccm)** | **Druck (mbar)** | **Leistung (W)** | **Zeit (s)** |
| **Beschichtung** | **HMDSO (Sccm)** | **O₂ (Sccm)** | | | | | | |
| Plasmapolymer U1 | 67 | 20 | 0,027 | 700 | 500 | 0,032 | 2000 | 10 |
| Plasmapolymer U2 | 67 | 20 | 0,027 | 700 | 500 | 0,032 | 2000 | 30 |

### Erläuterungen zu den XPS-Messungen

Die XPS-Untersuchungen wurden mit dem Spektrometer KRATOS AXIS Ultra der Fa. Kratos Analytical durchgeführt. Die Analysekammer war mit einer Röntgenquelle für monochromatisierte Al K_{α} - Strahlung, einer Elektronenquelle als Neutralisator und einem Quadrupolmassenspektrometer ausgerüstet. Weiterhin verfügte die Anlage über eine magnetische Linse, welche die Photoelektronen über einen Eintrittsschlitz in einen Halbkugelanalysator fokussierte. Durch Kalibrierung wurde der aliphatische Anteil des C 1s Peaks auf 285,0 eV gesetzt. Während der Messung zeigte die Oberflächennormale auf den Eintrittsschlitz des Halbkugelanalysators.

Die Passenergie betrug bei der Bestimmung der Stoffmengenverhältnisse jeweils 160 eV, die entsprechenden Spektren werden als Übersichtsspektren bezeichnet. Bei der Bestimmung der Detailspektren betrug die Passenergie jeweils 20 eV.

Die genannten Messbedingungen sind bevorzugt, um eine weitgehende Unabhängigkeit vom Spektrometertyp zu ermöglichen und um erfindungsgemäße plasmapolymere Produkte zu identifizieren.

Als Referenzmaterial wurde das Polydimethylsiloxan, Silikonöl DMS-T23E der Firma Gelest Inc. (Morrisville, USA) verwendet. Dieses trimethylsiloxy-terminierte Silikonöl besitzt eine kinematische Viskosität von 350 mm²/s (±10%) und eine Dichte von 0,970 g/ml bei 25 °C sowie ein mittleres Molekulargewicht von ca. 13.650 g/mol. Das ausgewählte Material zeichnet sich durch einen extrem geringen Anteil an verdampfbaren Bestandteilen aus: nach 24 Stunden bei 125 °C und 10⁻⁵ Torr wurden weniger als 0,01% flüchtige Anteile nachgewiesen (nach ASTM-E595-85 und NASA SP-R0022A). Es wurde mit Hilfe eines Spin-Coating-Prozesses als 40 bzw. 50 nm dicke Schicht auf einen Siliziumwafer aufgetragen; dabei wurde als Lösemittel Hexamethyldisiloxan verwendet.

Die Ergebnisse der beschriebenen XPS-Messungen sind in Tabelle 5 angegeben. Die atomare Zusammensetzung ist bezogen auf 100 Atom-% für die Summe der Elemente Silizium, Sauerstoff und Kohlenstoff. Der Gehalt an weiteren Elementen lag entweder unterhalb der Nachweisgrenze oder wie im Fall von Stickstoff bei unter 0,2%. Für das Curve Fitting wurden drei Signale mit den in Tabelle 1 angegebenen Bindungsenergien angenommen. Vergleichende Simulationen mit einem zusätzlichen Signal bei ca. 288,0 eV ergaben keinen signifikanten Anteil (also ein Anteil von > 1% an C 1s-Signalen) dieser Bindungsenergie, die beispielsweise charakteristisch für Aldehyd- und Keto-Gruppen ("C=O") wäre. Dies ist auch allgemein bevorzugt für die erfindungsgemäß zu verwendenden Schichten.

**Tabelle 5**

| **Plasma-polymer** | **atomare Zusammensetzung (at%)** | | | **Verhältnisse** | | | **Curve Fitting (% v. C-gesamt)** | | |
|---|---|---|---|---|---|---|---|---|---|
| | **O 1s** | **C 1s** | **Si 2p** | **C/Si** | **O/si** | **O/C** | **C** | **C-O** | **COO** |
| **A1** | 25,1 | 50,8 | 24,1 | 2,1 | 1,0 | 2,0 | 96,6 | 3,4 | 0,0 |
| **A6** | 49,7 | 23,0 | 27,4 | 0,8 | 1,8 | 0,5 | 93,3 | 6,7 | 0,0 |
| **U1** | 46,1 | 29,4 | 24,6 | 1,2 | 1,9 | 0,6 | 88,7 | 8,8 | 2,5 |
| **U2** | 58,7 | 15,1 | 25,2 | 0,6 | 2,3 | 0,3 | 73,4 | 18,5 | 8,2 |
| **B1** | 59,1 | 13,2 | 27,8 | 0,5 | 2,1 | 0,2 | 76,2 | 14,6 | 9,2 |

### Beispiel 2: Plasmapolymerbeschichtungen aus einem 250 I Reaktor

Die plasmapolymeren Beschichtungen B4 bis B9 (siehe unten) wurden mit einem Niederdruck-Plasmareaktor, der ein Volumen von 250 I (Beschreibung in ISBN 3-8265-9216-6 "Charakterisierung der spektroskopischen Eigenschaften von Metall- und Halbleiterclustern in plasmapolymeren Matrizen" von Dr. Dirk Salz) aufweist, durchgeführt. Die elektrische Anregungsspannung hat eine Frequenz von 13,56 MHz. Der Abstand zwischen der Plasmaentladungselektrode und dem Substrat (SiliziumWafer) betrug 30 cm. Vor jedem Experiment wurde der Niederdruck-Reaktor auf einen Druck von 0.01 mbar evakuiert. Das Restgas bei diesem Druck besteht zu mehr als 95% aus Wasserdampf. Die Beschichtungen wurden mit XPS untersucht. Die C1s-Spektren wurden einem Curve-Fitting unterzogen. Zudem wurde innerhalb von weniger als 12 Stunden auf den beschichteten Siliziumwafern der Kontaktwinkel von Wasser mit fortschreitendem Tropfen bei 25°C gemessen.

### Ausführungsbeispiel Plasmapolymer B4

Zwei Siliziumwafer wurden in drei Prozessschritten beschichtet. Im ersten Schritt wurde eine Sauerstofffeinreinigung durchgeführt, anschließend der Beschichtungsschritt mit Hexamethyldisiloxan und abschließend eine Sauerstoffaktivierung. Die genauen Beschichtungsparameter sind in Tabelle 6 angegeben. Es wurden Schichten mit einer Dicke von ca. 60 nm hergestellt. Der Kontaktwinkel von Wasser betrug 23°.

**Tabelle 6: Beschichtungsparameter des Ausführungsbeispiels Plasmapolymer B4**

| | Feinreinigung | Beschichtung | Aktivierung |
|---|---|---|---|
| O₂-Fluss / sccm | 60 | 60 | 60 |
| HMDSO-Fluss / sccm | 0 | 5 | 0 |
| Plasmaleistung / W | 800 | 800 | 800 |
| Behandlungszeit / min | 10 | 10 | 10 |
| Prozessdruck / mbar | 0,022 | 0,026 | 0,022 |

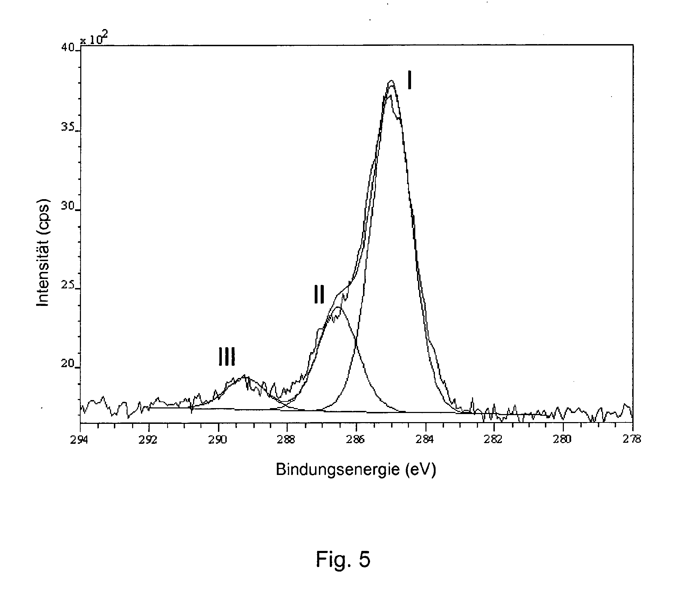

Die XPS-Analyse liefert die atomare Zusammensetzung Sauerstoff = 65,9%, Silizium = 27,0%, Kohlenstoff = 7,03%, Stickstoff = 0,12%. Das experimentell bestimmte hoch aufgelöste C1s-Spektrum zeigt Figur 5 als Einhüllende der drei einzelnen Gauß-Lorentz-Funktionen, die mit einem Curve-Fit bestimmt worden sind. Die Flächenquantifizierung ergibt folgendes Ergebnis für den Kohlenstoff: Aliphatischer Kohlenstoffanteil = 77,1% (285 eV), C-O-Gruppen = 16,5% (286,6 eV) und COO-Gruppen = 6,4% (289,3 eV). Die Zahlen in Klammern entsprechen dem energetischen Maximum der Gauß-Lorentz-Funktionen. Vergleichende Simulationen mit einem zusätzlichen Signal bei 288,0 eV ergaben einen Anteil dieser Bindungsenergie von unter 1% (bei diesem und nachfolgenden entsprechenden Simulationen selbstverständlich bezogen auf die Gesamtheit der C 1s-Signale).

### Ausführungsbeispiel Plasmapolymer B5

Dieses Experiment wurde wie Ausführungsbeispiel B4 durchgeführt, jedoch betrug die Aktivierungszeit 20 min, statt 10 min.

**Tabelle 7: Beschichtungsparameter des Ausführungsbeispiels Plasmapolymer B5**

| | Feinreinigung | Beschichtung | Aktivierung |
|---|---|---|---|
| O₂-Fluss / sccm | 60 | 60 | 60 |
| HMDSO-Fluss / sccm | 0 | 5 | 0 |
| Plasmaleistung / W | 800 | 800 | 800 |
| Behandlungszeit / min | 10 | 10 | 20 |
| Prozessdruck / mbar | 0,022 | 0,026 | 0,022 |

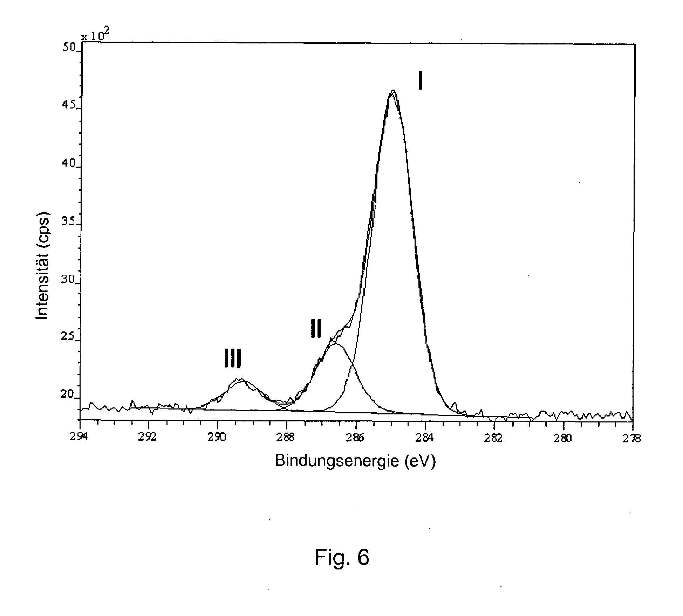

Die Schichtdicke und der Wasserkontaktwinkel auf den Si-Wafern betragen 60 nm bzw. 13°. Die XPS-Analyse liefert folgende atomare Zusammensetzung: Sauerstoff = 66,2%, Silizium = 27,4%, Kohlenstoff = 6,29%, Stickstoff = 0,13%. Das Ergebnis des Curve-Fits (Figur 6) liefert folgende Konzentrationen für den Kohlenstoff: Aliphatischer Kohlenstoffanteil = 76,8 % (285 eV), C-O-Gruppen = 16,4% (286,5 eV) und COO-Gruppen = 6,8% (289,2 eV). Vergleichende Simulationen mit einem zusätzlichen Signal bei 288,0 eV ergaben einen Anteil dieser Bindungsenergie von unter 1%.

### Ausführungsbeispiel Plasmapolymer B6

Dieses Experiment wurde wie Ausführungsbeispiel B4 durchgeführt, jedoch fand die Aktivierung mit Wasserdampf statt, welcher ständig aus den Reaktorwänden desorbiert wird.

**Tabelle 8: Beschichtungsparameter des Ausführungsbeispiels Plasmapolymer B6**

| | Feinreinigung | Beschichtung | Wasserdampfplasma |
|---|---|---|---|
| O₂-Fluss / sccm | 60 | 60 | 0 |
| HMDSO-Fluss / sccm | 0 | 5 | 0 |
| Plasmaleistung / W | 800 | 800 | 800 |
| Behandlungszeit / min | 10 | 10 | 10 |
| Prozessdruck / mbar | 0,022 | 0,026 | 0,022 |

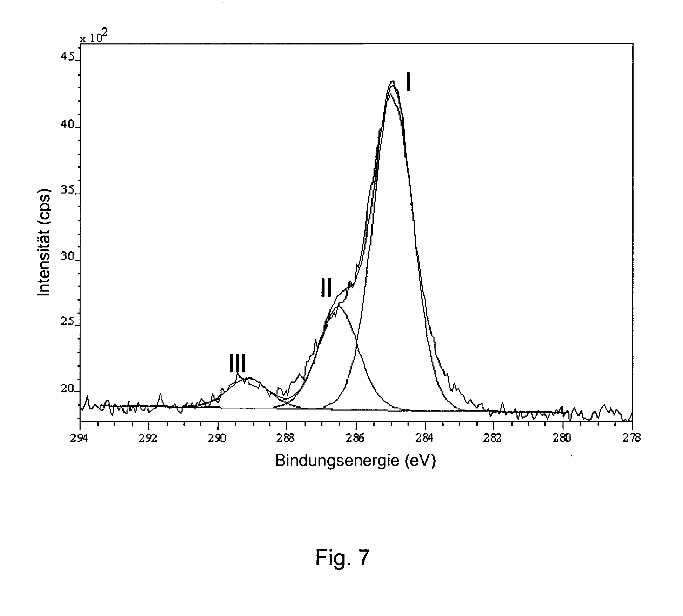

Die Schichtdicke und der Wasserkontaktwinkel auf den Si-Wafern betragen 60 nm bzw. 5°. Die XPS-Analyse liefert für die ersten 10 nm folgende atomare Zusammensetzung: Sauerstoff = 65,7%, Silizium = 27,2%, Kohlenstoff = 6,96%, Stickstoff = 0,12%. Das Ergebnis des Curve-Fits (Figur 7) liefert folgende Konzentrationen für den Kohlenstoff: Aliphatischer Kohlenstoffanteil = 73,4% (285 eV), C-O-Gruppen = 21,2% (286,5 eV) und COO-Gruppen = 5,4 % (289,1 eV). Vergleichende Simulationen mit einem zusätzlichen Signal bei 288,0 eV ergaben einen Anteil dieser Bindungsenergie von unter 1 %.

### Ausführungsbeispiel B7

Dieses Experiment wurde wie Ausführungsbeispiel B5 durchgeführt, jedoch fand die Aktivierung mit Wasserdampf 20 min lang statt, welcher ständig aus den Reaktorwänden desorbiert wird.

**Tabelle 9: Beschichtungsparameter des Ausführungsbeispiels Plasmapolymer B7**

| | Feinreinigung | Beschichtung | Wasserdampfplasma |
|---|---|---|---|
| O₂-Fluss / sccm | 60 | 60 | 0 |
| HMDSO-Fluss / sccm | 0 | 5 | 0 |
| Plasmaleistung / W | 800 | 800 | 800 |
| Behandlungszeit / min | 10 | 10 | 20 |
| Prozessdruck / mbar | 0,022 | 0,026 | 0,022 |

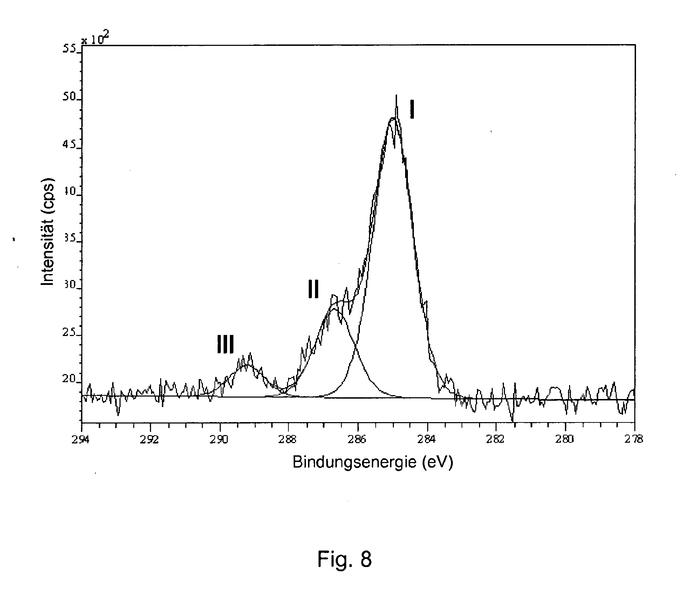

Die Schichtdicke und der Wasserkontaktwinkel auf den Si-Wafern betragen 60 nm bzw. annähernd 0 Grad. Die XPS-Analyse liefert für die ersten 10 nm folgende atomare Zusammensetzung: Sauerstoff = 65,5%, Silizium = 26,9%, Kohlenstoff = 7,44%, Stickstoff = 0,14%. Das Ergebnis des Curve-Fits (Figur 8) liefert folgende Konzentrationen für den Kohlenstoff: Aliphatischer Kohlenstoffanteil = 70,4 % (285 eV), C-O-Gruppen = 22,4 % (286,7 eV) und COO-Gruppen = 7.1 % (289,2 eV). Vergleichende Simulationen mit einem zusätzlichen Signal bei 288,0 eV ergaben einen Anteil dieser Bindungsenergie von unter 1 %.

### Ausführungsbeispiel Plasmapolymer B8

Für dieses Beispiel wurde Polyurethan (PU) mit einer hydrophilen ca. 40 nm dicken Plasmabeschichtung versehen. Der Wasserkontaktwinkel beträgt annähernd 0°. Die Beschichtungsparameter zeigt Tabelle 10 Die Bakterienadhäsion wurde mit dem Adhäsionsassay nach ISO 17025 bestimmt.

**Tabelle 10: Beschichtungsparameter des Ausführungsbeispiels Plasmapolymer B8**

| | Feinreinigung | Beschichtung | Aktivierung |
|---|---|---|---|
| O₂-Fluss / sccm | 60 | 400 | 100 |
| HMDSO-Fluss / sccm | 0 | 2.5 | 0 |
| Plasmaleistung / W | 800 | 2500 | 1000 |
| Behandlungszeit / min | 10 | 4 | 10 |
| Prozessdruck / mbar | 0.022 | 0.045 | 0.032 |

### Ausführungsbeispiel Plasmapolymer B9

Für dieses Beispiel wurde Polyurethan (PU) mit einer hydrophoben ca. 220 nm dicken Plasmabeschichtung versehen. Der Wasserkontaktwinkel beträgt annähernd 110°. Die Beschichtungsparameter zeigt Tabelle 11. Die Bakterienadhäsion wurde mit dem Adhäsionsassay nach ISO 17025 bestimmt.

**Tabelle 11: Beschichtungsparameter des Ausführungsbeispiels Plasmapolymer B9**

| | Feinreinigung | Beschichtung |
|---|---|---|
| O₂-Flus / sccm | 60 | 24 |
| HMDSO-Fluss / sccm | 0 | 70 |
| Plasmaleistung / W | 800 | 700 |
| Behandlungszeit / min | 10 | 10 |
| Prozessdruck / mbar | 0,022 | 0,034 |

### Beispiel 3: VUV-Bestrahlung einer siliziumorganischen Flüssigkeit und eines Plasmapolymers

Flache Glassubstrate (Objektträger) wurden einseitig zunächst wie in Beispiel 1 beschrieben mit der Biokompatibilitätsschicht Plasmapolymer A1 beschichtet. Anschließend wurden sie mit 270 nm flüssigem Polydimethylsiloxan-Silikonöl AK50^{®} (Fa. Wacker) versehen. Hierbei wurde das Silikonöl als Reinstoff mit Hilfe eines Aerosols aufgetragen. Danach wurde zum Maskieren die Hälfte der Glassubstrate durch eine 2 mm dicke Glasplatte abgedeckt und die andere Probenhälfte unter Stickstoffatmosphäre bei 1 bar mittels VUV-Strahlung der Wellenlänge 172 nm vernetzt. Hierfür wurde die gesamte Probe 6 Minuten bei einem Abstand von 10 mm von einer Excimerlampe (Hersteller: Radium Lampenwerk GmbH, Xeradex-Strahler, 172nm) mit einer Strahlungsleistungsdichte von ca. 0,08 W/cm² bestrahlt. Die nicht-maskierte Hälfte der Probe soll nachfolgend als Biokompatibilitätsschicht Excimer C1 bezeichnet werden.

Das unvernetzte Silikonöl unter der Maskierungsscheibe konnte ohne Probleme mit Isopropanol abgespült werden, so dass auf dieser Fläche die Biokompatibilitätsschicht Plasmapolymer A1 wieder freigelegt werden konnte.

Analog zur Biokompatibilitätsschicht Excimer C1 wurde das Silikonöl AK50^{®} auf mehrere Glassubstrate (Objektträger) mit einer mittleren Schichtdicke von 360nm mittels Aerosolverfahren appliziert. Unter Stickstoffatmosphäre und einem Prozessdruck von 1 bar wurde ein Muster bei einem Abstand von 10mm zur Lampe und einer Strahlungsleistungsdichte von ca. 0,08 W/cm² 900s bestrahlt (Xeradex-Strahler, 172nm). Die Strahlungsenergie betrug somit ca. 75 J/cm². Durch die VUV-Behandlung trat ein Schichtschrumpf um 40% auf 216nm ein. Diese Beschichtungsvariante soll nachfolgend als Biokompatibilitätsschicht Excimer C2 bezeichnet werden.

Weiterhin wurde ein zweites Muster ebenfalls unter Stickstoffatmosphäre und einem Prozessdruck von 1 bar bei einem Abstand von 30mm zur Lampe und einer Strahlungsleistungsdichte von ca. 0,05 W/cm² 300s bestrahlt. (Xeradex-Strahler, 172nm). Die Strahlungsenergie betrug ca. 15 J/cm². Durch die VUV-Behandlung tritt ein Schichtschrumpf um 25% auf 270nm ein. Diese Beschichtungsvariante soll nachfolgend als Biokompatibilitätsschicht Excimer C3 bezeichnet werden.

Zudem wurde eine Glasplatte mit der Beschichtung Plasmapolymer A1 5 Minuten bei einem Abstand von 10 mm mit einer Strahlungsleistungsdichte von ca. 0,08 W/cm² analog zu den Silikonölproben bestrahlt.bzw. mit einer Strahlungsenergie von ca. 25 J/cm² (Xeradex-Strahler, 172nm). Auch hier fand die Bestrahlung innerhalb einer Stickstoffatmosphäre bei einem Prozessdruck von 1 bar statt. Diese Beschichtungsvariante soll nachfolgend als Biokompatibilitätsschicht Excimer C4 bezeichnet werden.

Die Ergebnisse der XPS-Messungen der mit Hilfe einer Excimer-Lampe behandelten Probe C2 ist in Tabelle 12 angegeben. Vergleichende Simulationen mit einem zusätzlichen Signal bei 288,0 eV ergaben einen Anteil dieser Bindungsenergie von unter 1 %.

**Tabelle 12**

| **Excimer** | **atomare Zusammensetzung (at%)** | | | | **Verhältnisse** | | | **Curve Fitting (% v. C-gesamt)** | | |
|---|---|---|---|---|---|---|---|---|---|---|
| | **N 1s** | **O 1s** | **C 1s** | **Si 2p** | **C/Si** | **O/Si** | **C/O** | **C** | **C-O** | **COO** |
| **C2** | 0,9 | 65,4 | 6,4 | 27,2 | 0,2 | 2,4 | 0,10 | 66,8 | 18,2 | 15,0 |

### Beispiel 4: Biologische Beurteilung der Zelladhäsion

Im Folgenden wird ein Testverfahren beschrieben, mit dem die Biokompatibilitätsschichten biologisch beurteilt wurden. Es handelt sich hierbei um ein Färbeverfahren für an Oberflächen adhärierte Zellen, welches zu den Standardmethoden in der Zellbiologie zählt. Die auf der Oberfläche angewachsenen oder adhärenten Zellen werden hierbei mit einem Farbstoff direkt angefärbt und mikroskopisch beurteilt. Die Zahl der adhärierten Zellen kann hierbei als Zellen pro Fläche oder als Bedeckungsgrad in Prozent angegeben werden. Durch die mikroskopische Beurteilung der adhärierten Zellen ist zusätzlich möglich, die Zellen morphologisch zu beurteilen. Hierdurch kann, neben der Anzahl der Zellen pro Fläche, eine zusätzliche Beurteilung getroffen werden, wie die Biokompatibilitätsschicht auf die Zellen wirkt. Für die Betrachtung von Materialien, die nicht transparent sind, muss dies mit einem Auflichtmikroskop erfolgen (z.B. das Imager M1 Mikroskop von Zeiss).

Für die unten aufgeführten Testungen wurde die in der EN ISO 10993-5: 1999 für die Prüfung von Medizinprodukten u.a. empfohlene Zelllinie V-79 verwendet. Zum Anfärben der adhärierten Zellen wurde der in der medizinischen Histologie häufig verwendete Farbstoff Eosin-Hämatoxylin eingesetzt. Es können aber auch Vitalfarbstoffe wie z.B. das Neutralrot verwendet werden, die zusätzlich noch eine Aussage zur Vitalität der Zellen geben können. Durch die Menge des aufgenommenen bzw. gebundenen Farbstoffs kann zwischen lebenden, geschädigten und toten Zellen unterschieden werden. Die Verwendung von Eosin-Hämatoxylin zum Anfärben der Zellen hat den zusätzlichen Vorteil, dass dieser Farbstoff die Zellen nicht nur im sichtbaren Licht, sondern auch im Fluoreszenz-Bereich anfärbt. Mit Hilfe eines Fluoreszenzmikroskops können hierdurch auch solche Zellen sichtbar gemacht werden, die aufgrund der Substratbeschaffenheit nicht oder nur schlecht im Lichtmikroskop beurteilt werden können.

Die Versuche wurden wie nachfolgend beschrieben durchgeführt. Die Vorbereitung der Zellstammkultur erfolgte hierbei in Anlehnung an die EN ISO 10993-5: 99. Die V79-Zellen werden in 96 × 21 mm Zellkulturschalen, mit einer Wachstumsfläche von 60,1 cm³ (TPP® Techno Plastic Products AG) bei 37 °C, 5%-CO₂-Gehalt und wasserdampfgesättigter Atmosphäre im Brutschrank mit HAM's F12-Medium (10% Fötales Kälber Serum [FKS]) kultiviert. Bei etwa 80% Konfluenz (Bedeckung nach 3-4 Tage) erfolgte eine Passage der Zellen. Zur Herstellung einer Zellsuspension wurde zunächst das alte Medium aus den Schalen abgesaugt. Anschließend wurden die Schalen mit je 2 ml phosphat-gepufferter Salzlösung (PBS) gewaschen. Dann erfolgte ein erneutes Waschen mit 0,5 ml einer unverdünnten Trypsin-Lösung (vom Schweine-Pankreas, SIGMA-Aldrich) und Absaugen dieser Lösung. Anschließend wurden 3-4 Tropfen der unverdünnten Trypsin-Lösung auf die Zellen gegeben und für ca. 5 Minuten im Brutschrank inkubiert. Durch diese Behandlung lösen sich die Zellen von der Oberfläche ab und waren im Mikroskop als runde Zellen sichtbar. Um die enzymatische Reaktion abzustoppen, wurden 2 ml Serumhaltiges Kulturmedium hinzugegeben (HAM's F12).

Die Zellsuspension wurde durch vorsichtiges Auf- und Abpipettieren mit einer Pasteurpipette gut gemischt. Hierdurch wurde gewährleistet, dass sich auch die letzten Zellcluster auflösten. Anschließend erfolgte mit Hilfe einer Neubauer Zählkammer die Bestimmung der Zellen pro ml und die Einstellung der benötigten Zellzahl für die Versuche.

Die zu testenden, mit der Biokompatibilitätsbeschichtung versehenen Materialien wurden in die Vertiefungen einer 6-Lochzellkulturplatte (9,6 cm² Wachstumsfläche pro Vertiefung) überführt, wobei die Materialien vorher mit 70%igem Isopropanol, PBS und Nährmedium gewaschen wurden. Pro Vertiefung wurden dann 4 ml Zellsuspension mit 87.500 Zellen pro ml in Ham's F12 Medium mit 10% Serum hinzupipettiert. Pro Vertiefung waren somit insgesamt 350.000 V79 Zellen vorhanden. Anschließend wurden die Proben bei 37 °C, 5 %-CO₂-Gehalt und wasserdampfgesättigter Atmosphäre im Brutschrank für 24 h inkubiert.

Nach der Inkubation wurde das Nährmedium abgesaugt und die Proben mit je 2 ml PBS bei 50 Umdrehungen pro Minute 5 Minuten lang bei Raumtemperatur auf einem Orbitalschüttler gewaschen. Nach dem letzten Waschschritt wurde die Oberfläche der Proben mit je 1 ml einer Standard Eosin-Hämatoxylin Lösung bedeckt. Nach einer Inkubation von 3 Minuten bei 50 Umdrehungen pro Minute bei Raumtemperatur auf einem Orbitaischüttler wird die Färbelösung abgesaugt und die Proben zunächst 5 Minuten lang und danach 60 Minuten lang mit 2 ml PBS bei 50 Umdrehungen pro Minute bei Raumtemperatur gewaschen. Die Proben wurden anschließend mit Hilfe eines Auflichtmikroskops (Zeiss Axio Imager.M1) betrachtet und digitale Bilder (AxioCam MRC von Zeiss) aufgenommen. Für Substrate, die eine sehr dunkle Oberfläche besaßen und dadurch nur schlecht im Lichtmikroskop zu betrachten waren, wurde mit Hilfe von Fluoreszenzbeleuchtung und entsprechender Filtersätze - integrierte Bestandteile des Axio Imager.M1 Mikroskopes - Fluoreszenzbilder aufgenommen. Zur biologischen Beurteilung der Biokompatibilitätsschicht wurde zum einen die Morphologie der adhärierten Zellen bewertet. Bei einer die Zelladhäsion fördernden Oberfläche lagen die Zellen adhärent und gestreckt vor. Für Beschichtungen mit reduzierter Adhäsion lagen entweder keine Zellen vor oder solche, die eine abweichende Morphologie aufweisen. Zum anderen wurde mit Hilfe einer Bildverarbeitungssoftware (ImageJ) der Bedeckungsgrad berechnet. Bei einer Erhöhung des Bedeckungsgrades um 40 bis 80% im Vergleich zum unbehandelten Substrat liegt eine verbesserte Zelladhäsion vor, bei einer Erhöhung um mehr als 80% eine stark verbesserte Zelladhäsion. Bei einer Verringerung des Bedeckungsgrades um 40 bis 80% im Vergleich zum unbehandelten Substrat liegt eine reduzierte Adhäsion vor, bei einer Verringerung um mehr als 80% eine stark reduzierte Zelladhäsion.

**Tabelle 13: Bewertungsmatrix der Zelladhäsion mit der Zelllinie V-79**

| **Zelladhäsion** | **Änderung des Bedeckungsgrads** | **Morphologie** |
|---|---|---|
| verbessert | 40 % bis 80 % erhöht | adhärent, gestreckt |
| Stark verbessert | mehr als 80 % erhöht | adhärent, gestreckt |
| reduziert | 40 % bis 80 % verringert | kugelig-rund |
| Stark reduziert | mehr als 80 % verringert | kugelig-rund |

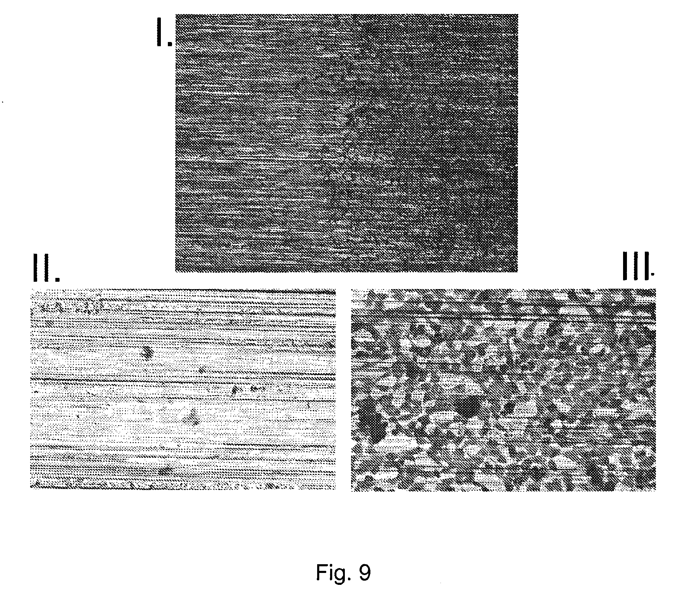

Die Ergebnisse der nachfolgend beschriebenen Versuche mit unterschiedlichen Materialien und Biokompatibilitätsbeschichtungen zeigen deutlich, dass mit Hilfe der Beschichtung unterschiedliche Oberflächeneigenschaften auf den Materialien erzielt werden können. Beispielhaft wird anhand der Figuren 9 bis 16 näher auf einige repräsentative Ergebnisse eingegangen.

Die Figur 9 zeigt unter I. ein Aluminiumplättchen, welches links mit der anti-adhäsiven Biokompatibilitätsschicht Plasmapolymer A1 und rechts mit der adhäsions-fördernden Biokompatibilitätsschicht Plasmapolymer B2 beschichtet wurde. Hierfür wurde zunächst Plasmapolymer A1 aufgebracht, dann die linke Hälfte der Probe mit einem Edelstahlplättchen abgedeckt (maskiert) und anschließend Plasmapolymer B2 aufgebracht. Unter II. wird ein Ausschnittsbild der linken Probenseite und unter III. ein Ausschnittsbild der rechten Probenseite gezeigt. Die adhärenten Fibroblastenzellen wachsen nur auf der rechten Seite. Die Morphologie der Zellen zeigt, dass diese adhärent und gestreckt vorliegen. Auf der mit einer anti-adhäsiven Beschichtung versehenen linken Oberfläche wachsen keine Zellen.

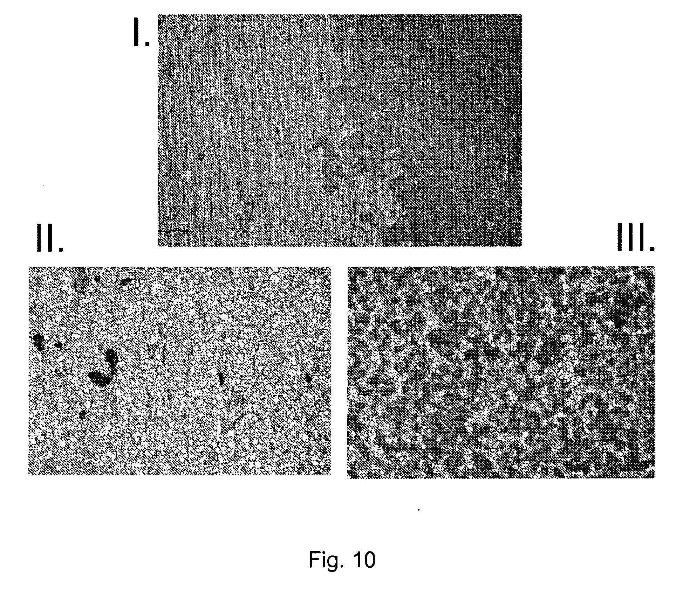

Die Figur 10 zeigt ein Substrat aus Edelstahl, welches links mit der anti-adhäsiven Biokompatibilitätsschicht Plasmapolymer A1 und rechts mit der adhäsions-fördernden Biokompatibilitätsschicht Plasmapolymer B2 beschichtet wurde. Hierfür wurde zunächst Plasmapolymer A1 aufgebracht, dann die linke Hälfte der Probe mit einem Edelstahlplättchen abgedeckt (maskiert) und anschließend Plasmapolymer B2 aufgebracht. Unter II. wird ein Ausschnittsbild der linken Probenseite und unter III. ein Ausschnittsbild der rechten Probenseite gezeigt. Die adhärenten Fibroblastenzellen wachsen nur auf der rechten Seite. Die Morphologie der Zellen zeigt, dass diese adhärent und gestreckt vorliegen. Die Oberfläche mit der Beschichtung für die reduzierte Zellanhaftung zeigt, dass hier nur sehr wenige Zellen zu sehen sind.

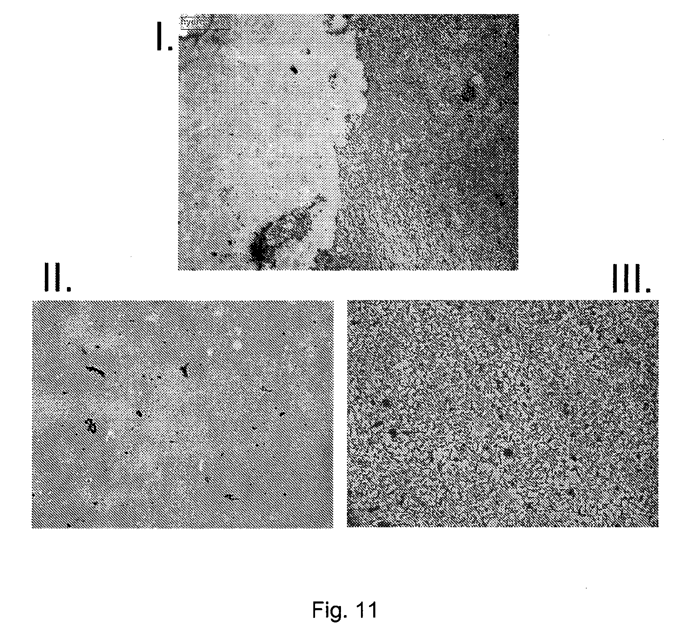

Die Figur 11 zeigt ein Glassubstrat, welches links mit der anti-adhäsiven Biokompatibilitätsschicht Plasmapolymer A1 und rechts mit der adhäsions-fördernden Biokompatibilitätsschicht Plasmapolymer B2 beschichtet wurde. Hierfür wurde zunächst Plasmapolymer A1 aufgebracht, dann die linke Hälfte der Probe mit einem Edelstahlplättchen abgedeckt (maskiert) und anschließend Plasmapolymer B2 aufgebracht. Hierdurch konnte ein unmittelbarer Übergang von Plasmapolymer A1 zu Plasmapolymer B2 realisiert werden, welcher in Figur 11 auf dem oberen Foto nach dem Anfärben der adhärierten Zellen zu sehen ist. Die adhärenten Fibroblastenzellen wachsen nur auf der rechten Seite mit dem Plasmapolymer B2, wie in der Ausschnittsvergrößerung III deutlich zu sehen ist. Die Morphologie der Zellen zeigt, dass diese adhärent und gestreckt vorliegen. Auf der linken Seite, dem Plasmapolymer A1, wie auch in der Ausschnittsvergrößerung Bild II zu sehen wachsen keine Zellen auf der Oberfläche.

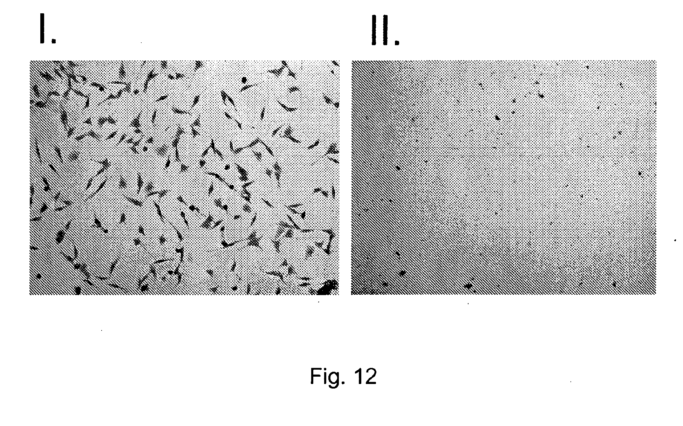

In Figur 12 sind zwei Bereiche einer Glasprobe zu sehen, welche zunächst mit der anti-adhäsiven Biokompatibilitätsschicht Plasmapolymer A1 beschichtet wurden. Anschließend wurde die Probe mit Silikonöl belegt, eine Hälfte mit einem Glas-Objektträger abgedeckt und die Probe wie oben beschrieben zur Erzeugung der Biokompatibilitätsschicht Excimer C1 mit Hilfe einer Excimerlampe vernetzt. Das Silikonöl unter dem Objektträger blieb unvernetzt und wurde mit Isopropanol abgewischt. Hierdurch resultierte eine Probe mit einer lokal abgegrenzten Fläche mit der anti-adhäsiven Biokompatibilitätsschicht Plasmapolymer A1, zu sehen in Bild II, und einer lokal abgegrenzten Fläche mit der Biokompatibilitätsschicht Excimer C1, zu sehen in Bild I. Auf der Fläche mit der freiliegenden anti-adhäsiven Biokompatibilitätsschicht Plasmapolymer A1 wachsen die Zellen gar nicht an. Auf der Fläche mit der Biokompatibilitätsschicht Excimer C1 wachsen die Zellen hingegen gut an.

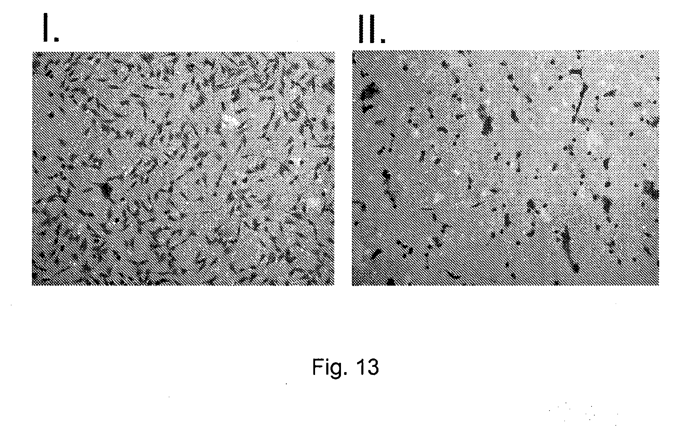

In Figur 13 sind Keramikproben zu sehen, in Bild I. unbehandelt und in Bild II. mit der anti-adhäsiven Biokompatibilitätsschicht Plasmapolymer A1 versehen. Auf der Probe in Bild II, die mit der anti-adhäsiven Beschichtung versehen ist, wachsen die Zellen reduziert an. Auf der unbehandelten Probe wachsen die Zellen hingegen gut an.

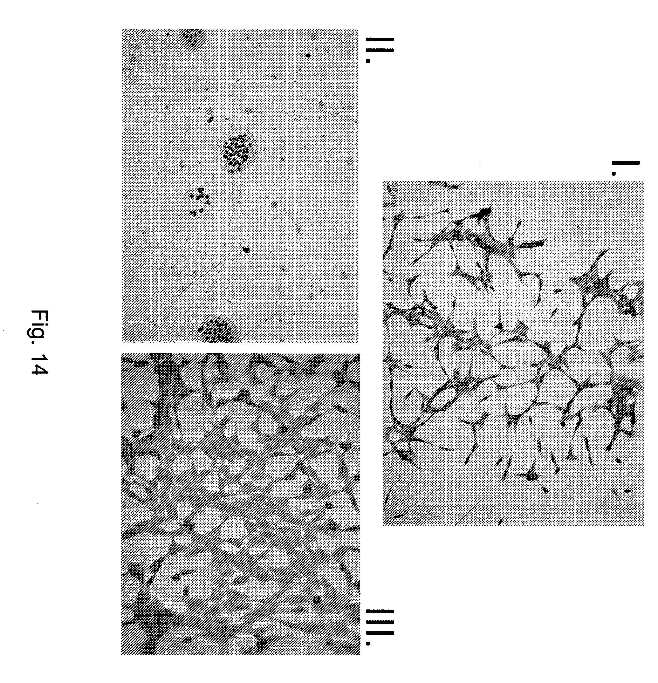

In Figur 14 sind PMMA-Kunststoffproben zu sehen, die mit unterschiedlichen Beschichtungen versehen wurden. Im oberen Bild I ist die unbehandelte Probe zu sehen, bei der die Zelladhäsion nur mittelmäßig ausgeprägt ist. In Bild III ist die mit der adhäsionsfördernden Biokompatibilitätsschicht Plasmapolymer B2 versehene Probe zu sehen; hier wachsen die Zellen verbessert an. Die Morphologie der Zellen zeigt, dass diese adhärent und gestreckt vorliegen. In Bild II. ist die mit der anti-adhäsiven Biokompatibilitätsschicht Plasmapolymer A1 versehene Probe zu sehen. Hier wachsen nur wenige bis keine Zellen.

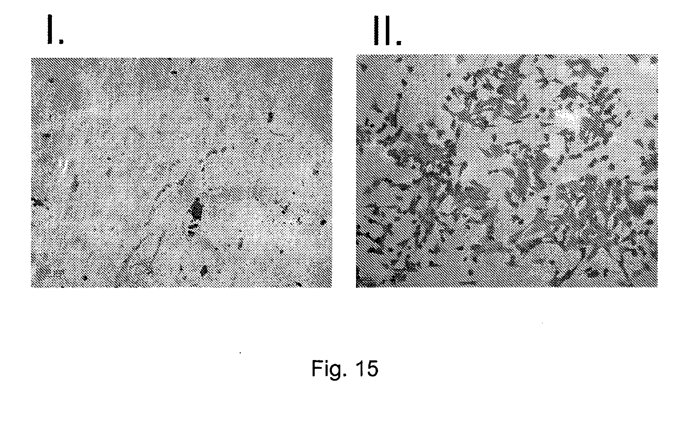

In Figur 15 sind Silikonproben zu sehen, in Bild I. die unbehandelte Referenz und in Bild II. mit der adhäsions-fördernden Biokompatibilitätsschicht Plasmapolymer B4 versehen. Auf der Probe Bild II., die mit der adhäsions-fördernden Beschichtung versehen ist, wachsen die Zellen verbessert an. Die Morphologie der Zellen zeigt, dass diese adhärent und gestreckt vorliegen. Auf der unbehandelten Probe Bild I. wachsen nur sehr wenige bis keine Zellen auf der Oberfläche.

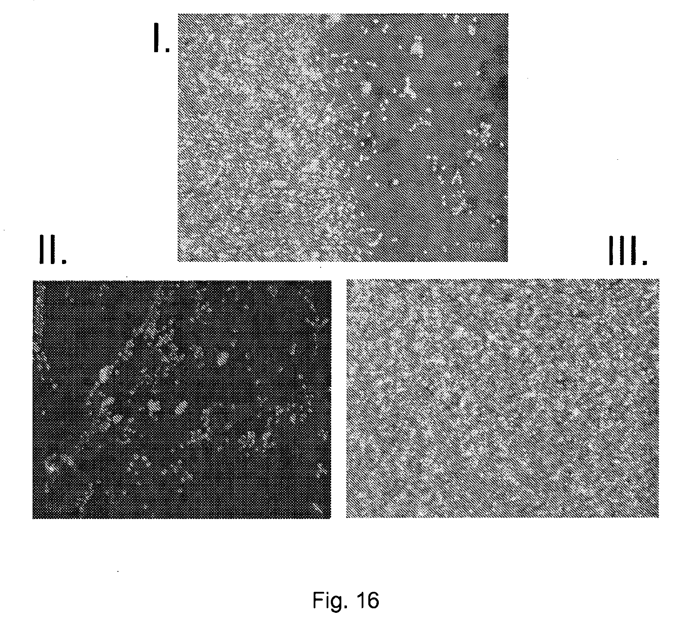

Die Figur 16 zeigt ein Substrat aus Titan, welches links unbehandelt ist (Bild I u. III) und rechts mit der anti-adhäsiven Biokompatibilitätsschicht Plasmapolymer A1 beschichtet wurde (Bild I u. II). Die Zellen wurden zur Sichtbarmachung im Fluoreszenzmikroskop betracht und das rote Farbbild in ein Graustufenbild umgewandelt.

Die Ergebnisse zur Zelladhäsionsuntersuchungen mit der Zelllinie V-79 sind in Tabelle 14 angegeben.

**Tabelle 14: Ergebnisse der Zelladhäsionsuntersuchungen mit der Zelllinie V-79 auf unterschiedlichen Materialien und Beschichtungen im Vergleich zu den unbehandelten Materialien**

| **Substrat** | **Beschichtung (Aus Beispiel 1, 2 oder 3)** | **Änderung der Zelladhäsion** |
|---|---|---|
| Aluminium | Plasmapolymer A1 | stark reduziert |
| Aluminium | Plasmapolymer B2 | nahezu gleich bleibend (gute Zelladhäsion) |
| Edelstahl | Plasmapolymer A1 | stark reduziert |
| Edelstahl | Plasmapolymer B2 | nahezu gleich bleibend (gute Zelladhäsion) |
| Glas | Plasmapolymer A1 | stark reduziert |
| Glas | Plasmapolymer A2 | stark reduziert |
| Glas | Plasmapolymer A3 | reduziert |
| Glas | Plasmapolymer A4 | stark reduziert |
| Glas | Plasmapolymer A5 | stark reduziert |
| Glas | Plasmapolymer A6 | stark reduziert |
| Glas | Plasmapolymer B2 | nahezu gleich bleibend (gute Zelladhäsion) |
| Glas | Plasmapolymer B3 | nahezu gleich bleibend (gute Zelladhäsion) |
| Glas | Excimer C1 | nahezu gleich bleibend (gute Zelladhäsion) |
| Keramik | Plasmapolymer A1 | stark reduziert |
| Keramik | Plasmapolymer B1 | nahezu gleich bleibend (gute Zelladhäsion) |
| Polymethylmethacrylat (PMMA) | Plasmapolymer A1 | stark reduziert |

| Polymethylmethacrylat (PMMA) | Plasmapolymer B2 | verbessert |
|---|---|---|
| Silikon | Plasmapolymer A1 | nahezu gleich bleibend (schlechte Zelladhäsion) |
| Silikon | Plasmapolymer B4 | stark verbessert |
| Titan | Plasmapolymer A1 | stark reduziert |

Analog zu den Die Ergebnisse zur Zelladhäsionsuntersuchungen mit der Zelllinie V-79 wurden auch Zelladhäsionsuntersuchungen mit der Zelllinie L-929 durchgeführt. Allerdings wurde hierbei zur Bewertung lediglich die Zellzahl herangezogen. Da die Untersuchungen sämtlich auf Glassubstraten erfolgten wurden die in Tabelle 15 aufgeführten Bewertungskriterien festgelegt.

**Tabelle 15: Bewertungsmatrix der Zelladhäsion mit der Zelllinie L-929**

| **Zelladhäsion** | **Änderung der Zellzahl pro Fläche gegenüber dem Glassubstrat** | |
|---|---|---|
| gut | um weniger als 45% verringert | |
| mittelmäßig | um 45% bis 60% verringert | |
| reduziert | um 61 % bis 80% verringert | |
| stark reduziert | mehr als 80 % verringert | |

Die Ergebnisse zur Zelladhäsionsuntersuchungen mit der Zelllinie **L-929** sind in Tabelle 15 angegeben.

**Tabelle 16: Ergebnisse der Zelladhäsionsuntersuchungen mit der Zelllinie L-929 auf unterschiedlichen Materialien und Beschichtungen im Vergleich zu den unbehandelten Materialien**

| **Substrat** | **Beschichtung (Aus Beispiel 1, 2 oder 3)** | **Änderung der Zelladhäsion** |
|---|---|---|
| Glas | Plasmapolymer A1 | stark reduziert |
| Glas | Plasmapolymer A6 | mittelmäßig |
| Glas | Plasmapolymer B1 | gut |
| Glas | Plasmapolymer U1 | mittelmäßig |
| Glas | Plasmapolymer U2 | gut |
| Glas | Excimer C2 | gut] |
| Glas | Excimer C3 | mittelmäßig |
| Glas | Excimer C4 | reduziert |

### Biologische Beurteilung der Bakterienadhäsion

Tabelle 17 zeigt die relative Bakterienadhäsion der Biokompatibilitätsschicht B8, bezogen auf unbeschichtetes Polyurethan (PU) mit definitionsgemäß 100% Adhäsion. Durch eine hydrophile Ausstattung der Oberfläche (Plasmapolymer B8) kann die Ädhäsion von gram-negativen E.Coli auf 66% gesenkt werden. Gleichzeitig nimmt sogar die Adhäsion von gram-positiven Staphylococcus epidermidis ab. Mit Hilfe der hydrophilen Beschichtung ist es möglich, dass bei beiden Bakterienarten die Adhäsion verringert wird.

**Tabelle 17: Relative Veränderung der Bakterienadhäsion**

| | Escherichia coli | Staphylococcus epidermidis |
|---|---|---|
| PU | 100% | 100% |
| PU+Beschichtung B8 | 66% | 83% |

Tabelle 18 zeigt die relative Bakterienadhäsion der Biokompatibilitätsschicht B9, bezogen auf unbeschichtetes PU mit definitionsgemäß 100% Adhäsion. Durch eine hydrophobe Ausstattung der Oberfläche (Plasmapolymer C 6) kann die Adhäsion von gram-positiven Staphylococcus epidermidis auf 56 % gesenkt werden. Die Adhäsion von E. Coli hingegen wird nicht wesentlich verringert.

**Tabelle 18: Relative Veränderung der Bakterienadhäsion**

| | Escherichia coli | Staphylococcus epidermidis |
|---|---|---|
| PU | 100% | 100% |
| PU+Beschichtung B9 | 92% | 56% |

### Beispiel 5: Testung auf Gentoxizität in Anlehnung an DIN EN ISO 10993-3

Die Biokompatibilitätsschichten A1, B1, U1 und U2 wurden mit dem Kometen-Testverfahren auf DNS-schädigende Wirkungen untersucht. Bei diesem Test wird das Erbgut (DNS - Desoxyribonukleinsäure) einzelner humaner Zellen elektrophoretisch aufgetrennt, nachdem die jeweiligen Zellen mit den Testoberflächen bzw. Testsubstanzen inkubiert wurden. Beim Vorliegen einer das Erbgut schädigenden Wirkung kann im Mikroskop bei den Zellen ein Schweif von DNS-Fragmenten neben einem Kopf von intakter DNA beobachtet werden (daher der Name Kometen-Testverfahren). Das Vorhandensein des Schweifes zeigt deutlich an, dass die Substanzen bzw. Oberflächen zu sogenannten Strangbrüchen innerhalb der DNS Moleküle führen. Die vielen kleinen aufgebrochenen Stränge formen in der elektrophoretischen Auftrennung eine Art Schweif, während die intakte DNA, bedingt durch ihre ursprüngliche Größe, die Form eines Kreises aufweist.

Für die Testung auf Gentoxizität wurde das Test-Kit "OxiSelect™" (Katalognummer STA-350) der Firma CELL BIOLABS, San Diego, USA verwendet (De Boeck et.al [2000], Validation and implementation of an internal standard in Comet assay. Mutat. Res. 469, 181-197).

Für den Test wurde die humane Zelllinie JURKAT eingesetzt. Als Negativkontrolle wurde eine unbehandelte Glasoberfläche verwendet. Eine Positivkontrolle wurde mit der Substanz Etoposid (Glycosid des Podophyllotoxins), einem Chemotherapeutikum, durchgeführt. Die Biokompatibilitätsschichten A1, B1, U1 und U2, aufgetragen auf Glaskörpern, und die Negativkontrolle (unbehandelte Glasoberfläche) wurden zusammen mit den Jurkat-Zellen für 24 Stunden bei 37°C und 5 % CO₂ im Brutschrank inkubiert. Als Zellkulturmedium wurde RPMI mit 10 % Serum und einer antibakteriellen Penicillin /Steptomycin-Lösung (2%) eingesetzt. Es wurden pro Versuch 300.000 Zellen pro mL auf die Testkörper gegeben. Für die Positivkontrolle wurde eine 100 µM Lösung Etoposid in Zellkulturmedium eingesetzt. Die Zellen, ohne Glasträger, wurden für 1 Stunde mit der Etoposid-Lösung im Brutschrank inkubiert (37°C, 5% CO₂). Bei den Jurkat-Zellen handelt es sich um sogenannte Suspensionszellkulturen, die nicht, im Gegensatz zu den adhärierenden Zellen, an den Oberflächen anwachsen, sondern nur auf der Oberfläche aufliegen. Für das Kometen-Testverfahren wurden die Zellen abgenommen und bei 700xg für 2 Minuten zentrifugiert und in phosphatgepufferter Salzlösung (PBS) resuspendiert und erneut zentrifugiert. Hiernach wurden die Zellen in einer entsprechenden Menge an PBS-Puffer aufgenommen, so dass eine Zellkonzentration von 100.000 Zellen pro Milliliter in PBS vorlag. Die Zellen wurden im nächsten Schritt im Verhältnis 1:10 mit niedrig schmelzender Agarose (37 °C) vermengt, gemischt und hiervon sofort 75 µL auf Glasträger (im Test Kit enthalten) pipettiert und vorsichtig ausgestrichen.

Im nächsten Schritt wurden die mit den Zellen beschichteten Glasträger mit Lysepuffer behandelt (14,6 g Natriumchlorid, 20 mL EDTA Lösung, 10 mL 10x OxiSelect Lysepuffer, mit destilliertem Wasser auf 100 mL aufgefüllt - pH 10) und für 50 Minuten bei 4°C im Dunklen inkubiert. Nachfolgend wird der Lysepuffer entfernt und durch einen alkalischen Puffer ersetzt (1,2 g Natriumhydroxid, 0,2 mL EDTA Lösung, mit destilliertem Wasser auf 100 mL aufgefüllt). Hiermit werden die Zellen für weitere 30 Minuten bei 4°C im Dunklen inkubiert.

Für die elektrophoretische Auftrennung der DNS bzw. der DNS-Fragmente wurde ein alkalisches Puffersystem verwendet (12,0 g Natriumhydroxid, 2 mL EDTA Lösung, mit destilliertem Wasser auf 1 Liter aufgefüllt). Hierfür wurden die mit den Zellen beschichteten Glasträger vorsichtig in eine Elektrophoresekammer gelegt und diese mit alkalischem Puffer befüllt, so dass die Glasträger knapp mit Puffer überdeckt waren. Die Elektrophorese wurde mit 300 mA und 25 Volt für 15 Minuten durchgeführt.

Die Glasträger mit den Zellen wurden anschließend dreimal mit destilliertem Wasser gewaschen. Nachfolgend wurden die Glasträger mit den Zellen für 5 Minuten mit 70%igem Ethanol inkubiert. Anschließend wurde das Ethanol abgesaugt und die Glasträger getrocknet. Zur Visualisierung der DNS wurden die Zellen in der Agarose auf dem Glasträger mit 100 µL des Fluoreszenzfarbstoffes Vista Grün DNA bei Raumtemperatur für 15 Minuten inkubiert. Die Zellen wurden mit einem Fluoreszenzmikroskop (Axio Imager M1 von Carl Zeiss Jena, Deutschland - Filtersatz für Anregung 495 nm und Emission 517 nm, Objektiv mit 20facher Vergrößerung) betrachtet. Es wurden für jeden Versuch etwa 50 Zellen zufällig ausgewählt und der Grad der Kometen-Bildung beurteilt.

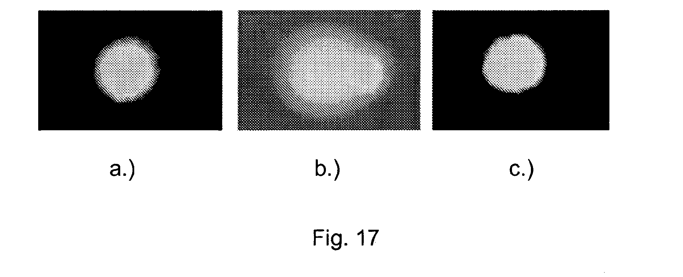

Die Negativkontrolle zeigte, dass weder durch das verwendete Glasträgermaterial, den Inkubationsbedingungen und verwendeten Lösungen, noch durch die verwendeten Zellen sowie durch einen falsch durchgeführten Kometen-Test eine erhöhte Rate von geschädigten Zellen zu beobachten war (siehe hierzu exemplarisch Figur 17.a). Die Bewertung der Negativkontrolle ergab weniger als 5 % Zellen mit einer Schädigung der DNS.

Die Positivkontrolle erlaubte einen Rückschluss darauf, wie eine gentoxische Substanz die DNS der Zelle schädigt und es im Kometen-Testverfahren zu einer Schweifbildung kommt (vergleiche exemplarisch Figur 17.b.). Die Behandlung der Zellen mit der Substanz Etoposid ergab eine starke gentoxische Schädigung der Zellen, wobei mehr als 95% der Zellen einen ausgeprägten Kometenschweif aufwiesen.

Zur Untersuchung einer möglichen gentoxischen Wirkung der erfindungsgemäß einzusetzenden Beschichtungen wurden diese ebenfalls mit dem Kometen-Testverfahren untersucht. Die Bewertung der Zellen ergab weniger als 5 % Zellen mit Kometenschweif und die Ergebnisse lagen damit im Bereich der Negativkontrolle. Dieses Resultat zeigt, dass die erfindungsgemäß einzusetzenden Beschichtungen den durchgeführten Experimenten keine gentoxischen Wirkungen aufweisen. Dieses Ergebnis lässt sich auf die erfindungsgemäß einzusetzenden Schichten insgesamt übertragen.

### Anwendungsbeispiele

### Anwendungsbeispiel 1: Oxigenatormembran

Polypropylen-Oxygenatorfasern wurden mit den hydrophilen Plasmapolymerschichten B4, B5, B6 und B7 beschichtet. Sauerstoffarmes Blut, welches in Kontakt mit den so beschichteten Fasern war, konnte Sauerstoff so effizient aufnehmen wie bei Verwendung der unbeschichteten Polypropylen-Oxygenator-Fasern. Anhand von Blutuntersuchungen konnte gezeigt werden, dass die Bildung von Thromben im Vergleich zu unbeschichteten Fasern deutlich reduziert worden ist.

### Anwendungsbeispiel 2: Implantate

Knochenfixationsnägel werden mit Bereichen mit reduzierter Zellanbindung (Plasmapolymerschicht A1) versehen, wie zu Figur 9 beschrieben maskiert, und anschließend mit Plasmapolymerschicht B2 versehen. Beide Bereiche (A1 und B2) werden wie in Beispiel 4 beschrieben auf ihre Biokompatibilität geprüft und als biokompatibel befunden. An der mit Plasmapolymer A1 beschichteten Fläche war die Zelladhäsion wie oben beschrieben gering, während sie an der mit B2 beschichteten Fläche wie beschrieben der von Edelstahl entsprach. Vergleichbare Befunde ergeben sich bei entsprechender Beschichtung von Hüft-, Knie-, Schulter- und Wirbelsäulenimplantaten, medizinischen Nägeln, Klammern, Fäden und/oder Schrauben. Beispielsweise sollten die Flächen im Inneren der Gelenkkapsel mit einer Biokompatibilitätschicht A ausgestattet werden, während die Bereiche, die Außerhalb der Gelenkkapsel liegen mit einer Biokompatibilitätschicht B ausgestattet werden.

### Anwendungsbeispiel 3: Gefäße (Arzneimittelbehältnisse) für Körperflüssigkeit, Gewebe, Biomoleküle, Pharmazeutika

Transport- bzw. Aufbewahrungsgefäße aus Glas für verdünnte wässrige Proteinlösungen können mit der Biokompatibilitätschicht Plasmapolymer A1 (gemäß Beispiel 1) beschichtet werden. Hierdurch wird eine deutliche Reduzierung der Anhaftung der Proteine erreicht. Dadurch kann zum einen die benötigte Ausgangskonzentration der Proteine deutlich reduziert werden. Zum anderen werden die Schwankungen in der realen Konzentration der Proteine verringert. Bei den Proteinen handelt es sich um therapeutisch wirksame Antikörper.

### Anwendungsbeispiel 4: Wundauflagen

Wundauflagen wurden auf eine Träger-Polymerfolie geklebt, welche in einem Bahnwarenprozess innerhalb des Plasmareaktors von Rolle zu Rolle gewickelt werden. Dabei wurde die Biokompatibilitätsschicht. Plasmapolymer A1 (gemäß Beispiel 1) aufgetragen. Die Schichtdicke auf Referenzsubstraten wurde mit ca. 50 nm bestimmt. Durch diese Plasmabeschichtung wird das Verkleben der Wundauflage durch während des Wundverschlusses neu gebildete Zellen vermindert, ein Gas- und Flüssigkeitsaustausch jedoch gewährleistet.

### Anwendungsbeispiel 5: Katheter (Blasenkatheter, Koronarkatheter)

Katheter werden im Bereich der außenliegenden Oberflächen in einem Plasmareaktor beschichtet. Hierzu werden die Katheter in einem Niederdruck-Plasmaprozess beschichtet. Bei Verwendung einer Biokompatibilitätsschicht Plasmapolymer A1 wird das Ein- und Ausführen der Katheter durch die veränderten Oberflächeneigenschaften verbessert.

### Langzeitwirkung

Bei Verwendung der Biokompatibilitätsschicht Plasmapolymer B2 (gemäß Beispiel 1) führt die Beschichtung dazu, dass der Katheter sich mit dem Gewebe besser verbinden lässt. Hierdurch kann z.B. erreicht werden, dass ein Langzeit-Katheter nicht so leicht verrutscht.

Zudem wurden Insulinkatheter aus Edelstahl mit der Biokompatibilitätsschicht Plasmapolymer A1 ausgestattet. Hierdurch kann das Zuwachsen des Insulinkatheters reduziert und somit die Standzeit des Katheters verlängert werden. Durch die Beschichtungen wird zudem die Hämokompatibilität erhöht.

### Anwendungsbeispiel 6: Angioplasieballon

Bei Verwendung einer Biokompatibilitätsschicht Plasmapolymer A1 (gemäß Beispiel 1) kann das Ein- und Ausführen des Angioplasieballon durch die veränderten Oberflächeneigenschaften erleichtert werden, da durch die veränderte Oberflächenspannung die Reibung vermindert werden kann. Dieses schont die bei diesem Eingriff betroffenen Gefäßareale.

### Anwendungsbeispiel 7: Blutgefäßstents

Blutgefäßstents können zunächst mit der Biokompatibilitätsschicht Plasmapolymer A1 (gemäß Beispiel 1) versehen werden. Anschließend kann die Außenseite mit Hilfe von UV-Strahlung oberflächlich oxidiert werden. Hierzu werden die Stents auf einem rotierenden Dorn bei einem Abstand von 10 mm unter einer Stickstoffatmosphäre bei einem Druck von 1 bar mit einem Feuchtigkeitsgehalt von ca. 1% mit dem Licht der Wellenlänge 172 nm einer Excimerlampe (Xeradex-Strahler, 172 nm, Radium Lampenwerk GmbH) ausgesetzt. Die Bestrahlung erfolgt beispielsweise über 30 Sekunden mit einer Strahlungsleistungsdichte von ca. 0,82 W/cm².

Durch diese Behandlung wird die Zelladhäsion auf der Innenseite des Stents deutlich verringert, während die Außenseite weiterhin eine gute Zelladhäsion ermöglicht. Durch die verbesserte Zelladhäsion auf der Außenseite kann der Spalt, der sich durch die Stentdilatation bildet, schneller geschlossen werden, da Zellen/ Gewebe auf dem Material anwachsen können. Eine innenliegende Behandlung der Oberfläche mit einer Biokompatibilitätsschicht des Typs A1 verhindert hingegen, dass sich Blutzellen bzw. Blutbestandteile an diesen Bereich anlagern und zu einer Verstopfung des Stents führen.

### Anwendungsbeispiel 8: Kanüle

Kanülen können mit der Biokompatibilitätsschicht Plasmapolymer A1 (gemäß Beispiel 1) versehen werden. Durch die Behandlung mit der Biokompatibilitätsschicht lassen sich die Kanülen leichter in Gewebe oder Gefäße platzieren bzw. ebenso leichter wieder entfernen.

### Anwendungsbeispiel 9: Injektionssystem

Kanülen können mit der Biokompatibilitätsschicht Plasmapolymer B2 (gemäß Beispiel 1) versehen werden. Durch die Behandlung mit der Biokompatibilitätsschicht wird erreicht, dass sich im Bereich des Gewebedurchtritts die Zellen besser an das Injektionsmaterial anlagern. Hierdurch wird eine verbesserte Wundheilung/ Verschluss erreicht. Das Eindringen von Krankheitserregern kann hierdurch stark reduziert werden.

### Anwendungsbeispiel 11: künstliches Organ

Als Transplantate werden z.B. künstliche Nieren oder Herzen seit einiger Zeit eingesetzt. Ein schwerwiegendes Problem ist hierbei, dass der Körper stark auf z.B. den Kunststoff dieser Transplantate reagiert. Durch eine Biokompatibilitätsschicht wie beispielsweise Plasmapolymer B2 können hierbei diese starken körpereigenen Reaktionen vermindert werden. Auf der anderen Seite ist es durch eine Biokompatibilitätsschicht wie beispielsweise Plasmapolymer A1 möglich zu verhindern, dass bestimmte Bereiche der künstlichen Organe durch ungewolltes Zellwachstum in ihrer Funktion beeinträchtigt werden.

### Anwendungsbeispiel 12: Herzschrittmacher und eine Stromquelle desselben

Herzschrittmacher können zusammen mit Elektroden, Kabeln und Gehäusen zunächst mit einer Biokompatibilitätsschicht wie beispielsweise Plasmapolymer A1 (gemäß Beispiel 1) ausgestattet werden. Anschließend werden die Elektroden und Kabel mit Hilfe eines Sauerstoff-haltigen Plasmas aus einer Atmosphärendruck-Plasmadüse wie beispielsweise der Plasmadüse PFW 10 der Firma PlasmaTreat oberflächlich oxidiert. Zur Erzeugung eines Plasma-Strahls wird beispielsweise Luft mit einem Volumenstrom von ca. 1400 L/h durch die Plasmadüse geleitet und innerhalb der Düse ein gepulstes Plasma (Pulsdauer ca. 50 µs, Puls/Pause-Verhältnis ca. 1/3) mit einer Frequenz von ca. 18 kHz, einer Elektrodenspannung von ca. 10 kV und einer Plasmaleistung von ca. 0,8 kW erzeugt. Für die Behandlung wird beispielsweise ein Abstand von ca. 10 mm zwischen der Plasmadüse und der Oberfläche eingestellt. Die Behandlung erfolgt beispielsweise mit einer Relativgeschwindigkeit (zwischen Oberfläche und Düse) von 16 m/min.

Hierdurch kann die Verankerung der Elektroden und Kabel im Gewebe verbessert werden. Zudem wird es zu einer Verminderung des Einwachsens des Gehäuses durch umliegendes Gewebe kommen und die Biokompatibilität z.B. der Elektroden erhöht.

### Anwendungsbeispiel 13: Prothese (offene Implantate, Epithesen)

Prothesen, die vollständig von Gewebe umschlossen sind (so genannte Endoprothesen), werden im Anwendungsbeispiel 2 "Implantate" aufgeführt. Bei so genannten offenen Implantaten befindet sich ein Teil im Körpergewebe, während sich der andere Teil außerhalb befindet. Im Bereich des Durchtritts durch die Haut bzw. das Gewebe erhöht eine Biokompatibilitätsschicht des Typs Plasmapolymer B2 den Wundverschluss, da hier die Gewebezellen verbessert anwachsen. Durch diese Beschichtung wird zusätzlich die Gewebeverträglichkeit der offenen Prothese verbessert. Zusätzlich wird hierdurch verhindert, dass Bakterien über die Durchtrittsbereiche in den Körper gelangen und Infektionen auslösen, da diese Bereiche einen verbesserten Verschluss zwischen Gewebe und Prothesenmaterial haben.

### Anwendungsbeispiel 14: Cochlea -Implantat

Im Bereich der Cochlea-Implantate können Biokompatilitätsbeschichtungen des Typs Plasmapolymer B2 der dünnen Anschlusskabel dabei helfen, dass diese besser am Felsenbein fixiert werden. Dieses hilft dabei, dass das Elektrodenset nicht so leicht herausrutscht. Zusätzlich sorgt diese Beschichtung dafür, dass sich das Implantat besser in das umgebende Gewebe integriert. Hierdurch können u.a. Unverträglichkeiten gegenüber den Implantatmaterialien (z.B. dem Silikon) verhindert werden, welches einen schnelleren Heilungsprozess ermöglicht und Entzündungsprozesse verhindert.

### Anwendungsbeispiel 15: künstliche Herzklappe

Durch die Beschichtung der künstlichen Klappenoberfläche mit einer Biokompatibilitätsschicht Plasmapolymer A1 (gemäß Beispiel 1) kann die Neigung zur Bildung von Thromben reduziert werden.

### Anwendungsbeispiel 16: Herzklappenring

Durch eine Beschichtung des Herzklappenrings mit einer Biokompatibilitätsschicht Plasmapolymer B2 kann dieser verbessert in das umliegende Herzgewebe integriert werden.

### Anwendungsbeispiel 17: Intraocularlinse

Durch die Beschichtung einer Intraocularlinse mit einer plasmapolymeren Beschichtung des Typs B2 kann die Biokompatibilität erhöht werden, hierdurch wird das Einwachsen und die Benetzbarkeit der Intraocularlinse verbessert.

### Anwendungsbeispiel 19: Blutkonserven-Beutel

Beim Transport oder der Lagerung von Blut und Blutbestandteilen muss verhindert werden, dass diese mit der Oberfläche des Beutelmaterials reagieren, da hierdurch unerwünschte Reaktionen ausgelöst werden könnten. Durch die Behandlung des innenliegenden Beutelmaterials mit einer Biokompatibilitätsschicht Plasmapolymer A1 kann eine solche unerwünschte Reaktion vermindert werden.

### Anwendungsbeispiel 20: Zellkultur-Behältnis

Durch die Behandlung von Zellkulturschalen (Petrischalen, 6-, 12-, 24- und 96-Loch Platten) mit einer Biokompatibilitätsschicht des Typs B2 kann erreicht werden, dass die Zellen besser der Zellkultur-Behältnisoberfläche anwachsen. Dies hat entscheidende Vorteile für zellbiologische und Zytotoxizitäts Versuche.

Durch eine Biokompatibilitätsschicht Plasmapolymer A1 kann, bei z.B. Versuchen mit Blutzellen, verhindert werden, dass eine unerwünschte Reaktion dieser Zellen mit dem Testgefäß stattfindet, wie z.B. eine ungewollte Immunreaktion.

### Anwendungsbeispiel 21: Fermenter

Im Bereich der Fermenter kann eine Biokompatibilitätsschicht des Typs B2 dazu dienen, den Bewuchs von Oberflächenstrukturen im Fermenter zu verbessern. So lassen sich hierdurch zum Beispiel Zellen, die z.B. bestimmte Proteine bilden und in das diese Zellen umgebende Medium abscheiden, auf den Fermenteroberflächenmaterialien (Waben, Lamellen, 3D-Strukkturen u.a.) verbessert anlagern. Bei diesen Zellen handelt sich um Protein-produzierende CHO-Zellen. Hierdurch kann die Menge an gebildeter Protein-Substanz erhöht werden.

### Anwendungsbeispiel 26: Sensor

Auf Sensoren, die mit einer Biokompatibilitätsschicht Plasmapolymer A1 versehen sind, werden sich im Bereich der Sensorfläche weniger bzw. keine unerwünschten Zellen oder Biomoleküle anlagern. Durch eine Ablagerung im Bereich des Sensors kommt es häufig zum Versagen dieser Messinstrumente. Durch eine Biokompatibilitätsschicht Plasmapolymer A1 wird eine verlängerte Funktionsfähigkeit dieser Sensoren erreicht.

### Anwendungsbeispiel 27: Sonden

Perkutane Endoskopische Gastrostomie Sonden (oder weitere wie z.B die Perkutane Endoskopische Jejunostomie-Sonde) dienen der Versorgung von Patienten mit Nahrung und Flüssigkeiten. Im Bereich des Durchtritts durch den Magen bzw. Darm kann es zu Undichtigkeiten kommen, die dazu führen, dass sich der Magen/Darm-Inhalt in den Bauchraum ergießt und es zu lebensbedrohlichen Bauchfellentzündungen kommt. Durch eine Biokompatibilitätsschicht des Typs B2 kann sich das Magen/Darmgewebe verbessert an das Sondenmaterial anlagern und hierdurch Undichtigkeiten verringert werden.

### Anwendungsbeispiel 30: Filtermaterial

Durch die Beschichtung von Filtermaterial mit Hilfe einer Biokompatibilitätsschicht Plasmapolymer B2 kann erreicht werden, dass sich hier Bakterienzellen verbessert anheften können. Die hier angehefteten Zellen können dazu verwendet werden, z.B. bestimmte Substanzen wie organische Kontaminationen aus einer diese Zellen umströmenden Flüssigkeit oder Gasen herauszufiltern bzw. abzubauen. Anwendungen als Biofilter können hierdurch ermöglicht werden.

### Anwendungsbeispiel 31: Biokompatible Trägermatrices (engl. scaffolds)

Im Bereich der in vitro Gewebezüchtung werden zunehmend Trägermaterialien auf Basis von Keramiken oder Polymermaterialien verwendet. Für diese Trägermaterialien ist von zentraler Bedeutung, dass autologe Zellen auf diesen Trägermaterialien anwachsen. Dies kann beispielsweise mit der Biokompatibilitätsschicht Plasmapolymer B2 erleichtert werden.

### Anwendungsbeispiel 32: Künstliche Hornhaut (Cornea)

Verletzte oder erkrankte Hornhäute werden heutzutage vielfach durch das Transplantieren von Spender-Hornhäuten ersetzt. Allerdings sorgen der Mangel an geeigneten Spender-Hornhäuten sowie das Risiko der erneuten Erkrankung der implantierten Hornhäute für einen großen Bedarf an künstlichen Hornhäuten.

Um ein Einwachsen des Randes der künstlichen Hornhäute in das augeneigene Gewebe zu ermöglichen wird dieser Rand mit der Biokompatibilitätsschicht Plasmapolymer B2 beschichtet. Als Ergänzung kann der mittlere Bereich der künstlichen Hornhaut mit der Biokompatibilitätsschicht Plasmapolymer A1 ausgestattet werden, um hier das Absetzen von Zellen, die das Sehvermögen herabsetzen würden, zu verhindern.

Alternativ wird die gesamte künstliche Hornhaut zunächst mit der Biokompatibilitätsschicht Plasmapolymer A1 ausgestattet und anschließend nach Maskierung des mittleren Teils der Rand mit Hilfe einer Plasmaaktivierung oder durch Excimerstrahlung in Gegenwart von zumindest Spuren von Sauerstoff oberflächlich oxidiert.

Zur weiteren Verbesserung des Einwachsens des Randbereiches können spezielle Proteine auf die erfindungsgemäße Beschichtung aufgebracht werden.

## Patentansprüche

1. Verwendung einer vernetzten siliziumhaltigen Schicht enthaltend, bestehend im wesentlichen aus oder bestehend aus Silizium, O, C, H, optional N, die durch Plasmapolymerisation und/oder Vernetzung von siliziumorganischen Flüssigkeiten mit Hilfe eines Plasmaprozesses und/oder UV-Strahlung einer Wellenlänge unter 250 nm ohne die Verwendung von Metallen einer Ordnungszahl über 14 herstellbar ist, mit einem atomaren Verhältnis von Sauerstoff zu Silizium von 0,75 bis 2,2 und einem atomaren Verhältnis von Kohlenstoff zu Silizium von 0,1 bis 2,5, gemessen mit XPS.
- als nicht-genotoxische Oberfläche oder
- zum Vermitteln oder Versehen einer Oberfläche mit einer nicht-genotoxischen Wirkung.

2. Verwendung nach Anspruch 1, wobei die Schicht außerdem zur definierten (i) Verbesserung oder (ii) Herabsetzung der Adhäsion von Biomolekülen und/oder Zellen (Pro - oder Eukaryonten) verwendet wird.

3. Verwendung einer vernetzten siliziumhaltigen Schicht enthaltend, bestehend im wesentlichen aus oder bestehend aus Silizium, O, C, H, optional N, die durch Plasmapolymerisation und/oder Vernetzung von siliziumorganischen Flüssigkeiten mit Hilfe eines Plasmaprozesses und/oder UV-Strahlung einer Wellenlänge unter 250 nm ohne die Verwendung von Metallen einer Ordnungszahl über 14 herstellbar ist, mit einem atomaren Verhältnis von Sauerstoff zu Silizium von 0,75 bis 2,2 und einem atomaren Verhältnis von Kohlenstoff zu Silizium von 0,1 bis 2,5, gemessen mit XPS zur definierten (i) Verbesserung oder (ii) Herabsetzung der Adhäsion von Biomolekülen und/oder Zellen (Pro- oder Eukaryonten) oder
Verwendung nach Anspruch 2,
jeweils mit der Maßgabe, dass für den Fall, dass eine Herabsetzung der Adhäsion von Biomolekülen und/oder Zellen durch die Verwendung erfolgt, dass die vernetzte siliziumhaltige Schichtnicht aus Kohlenstoff, Silicium, Sauerstoff und Wasserstoff sowie gegebenenfalls üblichen Verunreinigungen besteht, wobei im ESCA-Spektrum der Schicht, bei Kalibrierung auf den aliphatischen Anteil des C 1s Peaks bei 285,00 eV, im Vergleich mit einem trimethylsiloxy-terminierten Polydlmethylsiloxan (PDMS) mit einer kinematischen Viskosität von 350 mm²/s bei 25 °C und einer Dichte von 0,97 g/mL bei 25 °C.
der Si 2p Peak einen Bindungsenergiewert besitzt, der um maximal 0,45 eV zu höheren oder niedrigeren Bindungsenergien verschoben ist, und
der O 1s Peak einen Bindungsenergiewert besitzt, der um maximal 0,50 eV zu höheren oder niedrigeren eindungsenergien verschoben ist.

4. Nicht-genotoxischer medizintechnischer Gegenstand, umfassend einen Oberflächenbereich mit einer vernetzten siliziumhaltigen Schicht enthaltend, bestehend im wesentlichen aus oder bestehend aus Silizium, O, C, H, optional N, die durch Plasmapolymerisation und/oder Vernetzung von siliziumorganlschen Flüssigkeiten mit Hilfe eines Plasmaprozesses und/oder UV-Strahlung einer Wellenlänge unter 250 nm ohne die Verwendung von Metallen einer Ordnungszahl über 14 herstellbar ist, mit einem atomaren Verhältnis von Sauerstoff zu Silizium von 0,75 bis 2,2 und einem atomaren Verhältnis von Kohlenstoff zu Silizium von 0,1 bis 2,5, gemessen mit XPS, wobei der Gegenstand ausgewählt ist aus der Gruppe bestehend aus:
a) Membran, Rohr und Schlauch, insbesondere Oxygenatormembran, Katheter, Angioplasieballon, Stent, Kanüle, Sensor und Sonde,
b) einem bei bestimmungsgemäßem Gebrauch implantierten Gegenstand, ausgewählt aus der Gruppe bestehend aus:
medizinischer Nagel, Klammer, Faden und Schraube, insbesondere Knochenfixationsnagel, Stent oder Gefäßprothese, Injektionssystem, Katheter, kardiovaskutäres Implantat, künstliches Organ, insbesondere Herzschrittmacher und Stromquelle desselben, Prothese, orthopädisches Implantat, insbesondere künstlicher Gelenkkörper, insbesondere Gelenkpfanne und damit zusammenwirkendes Gegenstück, wie Hüft- oder Knieprothese, Wirbelsäulenprothese, Cochlea-implantat, künstliche Herzklappe, Herzklappenring oder Intraocularlinse, künstliche Hornhaut. Pumpen oder andere Vorrichtung zur Freisetzung von Substanzen im Körper und Epithese,
c) einem Behältnis zur Aufnahme und/oder zum Transport von Körperflüssigkeit, Gewebe oder deren Bestandteilen eines Lebewesens oder von Biomolekülen, bevorzugt Peptiden, Proteinen, Lipiden, Kohlenhydraten, Nukleinsäuren oder damit hergestellten Wirkstoffen.
d) einem Gegenstand zum zumindest teilweisen Bedecken von Haut oder Schleimhaut eines Lebewesens und bevorzugt von Wunden,
e) einem bei bestimmungsgemäßem Gebrauch anderweitig mit Körperflüssigkeit, Gewebe oder deren Bestandteilen eines Lebewesens oder mit Biomolekülen, bevorzugt Peptiden, Proteinen, Lipiden, Kohlenhydraten, Nukleinsäuren oder damit hergestellten Wirkstoffen in Kontakt stehendem Gegenstand,
**dadurch gekennzeichnet, dass** die Kohlenstoff-Atome der siliziumhaltigen Schicht einen Anteil von 5 - 35 % an Kohlenstoff-Atomen mit einer Bindung zu einem Sauerstoff-Atom ("C-O-Kohlenstoff") aufweisen, gemessen mit XPS und/oder
dass die Kohlenstoff-Atome der siliziumhaltigen Schicht einen Anteil von 5 - 20 % an Kohlenstoff-Atomen mit Bindungen zu zwei Sauerstoff-Atomen ("COO-Kohlenstoff") aufweisen, gemessen mit XPS.

5. Gegenstand nach Anspruch 4, **dadurch gekennzeichnet, dass** der Gegenstand einen Bereich der siliziumhaltigen Schicht besitzt mit
a) einem Wasser-Kontaktwinkel von nicht mehr als 35°. oder
b) einem Wasser-Kontaktwinkel von zumindest 90°.

6. Gegenstand nach einem der Ansprüche 4 bis 5, **dadurch gekennzeichnet, dass** die siliziumhaltige Schicht außer aus Wasserstoff zumindest zu 98 Atom-% aus den Elementen Silizium, Kohlenstoff und Sauerstoff und bevorzugt aus 0,1% bis zu 2% Stickstoff gemessen mit XPS besteht.

7. Gegenstand nach Anspruch 6, **dadurch gekennzeichnet, dass** die siliziumhaltige Schicht 3-15 Atom-% Kohlenstoff aufweist, bezogen auf alle Elemente außer Wasserstoff, bestimmt durch XPS.

8. Gegenstand nach einem der Ansprüche 4 bis 7, **dadurch gekennzeichnet, dass** die siliziumhaltige Schicht hergestellt oder herstellbar ist durch Vernetzen eines Methylsiloxan-Precursors, vorzugsweise von Hexamethyldisiloxan durch Plasmapolymerisation, insbesondere Niederdruck- oder Atmosphärendruck-Plasmapolymerisation, oder durch Vernetzen eines Silikonöls ohne chemisch reaktive Gruppen unter Einwirkung eines Plasmas oder UV-Strahlung einer Wellenlänge unter 250 nm, insbesondere Excimer-Strahlung.

9. Gegenstand nach Anspruch 8, **dadurch gekennzeichnet, dass** die siliziumhaltige Schicht nach dem Vernetzen des Precursors bzw. Silikonöls zumindest teilweise oxidiert wurde, vorzugsweise durch Plasma-Einwirkung, Beflammung, Oxy-Fluorierung, Laser-Behandlung oder Behandlung mit Excimer-Lampen.

10. Gegenstand nach einem der Ansprüche 4 bis 9, **dadurch gekennzeichnet, dass** die siliziumhaltige Schicht eine Schichtdicke von höchstens 2 µm, bevorzugt höchstens 1 µm, besonders bevorzugt höchstens 500 nm besitzt, sowie mindestens 5 nm, bevorzugt mindestens 10 nm, besonders bevorzugt mindestens 15 nm besitzt.

11. Verfahren zum Herstellen eines Gegenstandes nach einem der Ansprüche 4 bis 10, umfassend die Schritte
a) Bereitstellen einer vernetzten siliziumhaltigen Schicht mit einem atomaren Verhältnis von Sauerstoff zu Silizium von 0,75 bis 2.2, und einem atomaren Verhältnis von Kohlenstoff zu Silizium von 0,1 bis 2,5, gemessen mit XPS, und
b) zumindest abschnittweises Oxidieren der in Schritt a) bereitgestellten Schicht.

12. Verfahren nach Anspruch 11, **dadurch gekennzeichnet, dass** zum Begrenzen des zu oxidierenden Bereichs eine Maske, vorzugsweise eine ablösbare selbstklebende Maske, besonders bevorzugt ein Klebeband, oder eine gedruckte Maske und/oder ein vorzugsweise in Wasser zumindest teilweise lösbarer oder dispergierbarer Stoff verwendet und nach Schritt b) vorzugsweise entfernt wird.

13. Verwendung nach einem der Ansprüche 1 bis 3 zum im Vergleich zu einer entsprechenden unbeschichteten Oberfläche,
- Reduzieren einer Anhaftung pathogener Ablagerungen und/oder
- Reduzieren der Thrombogenität und/oder
- Reduzieren des Auftretens humoraler und zellulärer Immunreaktionen und/oder
- Reduzieren von unspezifischer Adsorption von Peptiden, Proteinen, Lipiden, Kohlenhydraten und/oder Nukleinsäuren an der Oberfläche und/oder
- Ermöglichen räumlich begrenzten Wachstums von Zellkulturen auf der Oberfläche und/oder.
- Erhöhen der Benetzbarkeit durch wässrige Flüssigkeiten, insbesondere Blut.

## Claims

1. Use of a cross-linked silicon-containing layer containing, consisting substantially of or consisting of silicon, O, C, H, optionally N, which can be produced by plasma polymerisation and/or cross-linking of organo-silicon liquids with the aid of a plasma process and/or UV radiation of a wavelength of less than 250 nm without the use of metals of an atomic number above 14, with an atomic ratio of oxygen to silicon of 0.75 to 2.2 and an atomic ratio of carbon to silicon of 0.1 to 2.5, measured with XPS,
- as a non-genotoxic surface, or
- for mediating or providing a surface with a non-genotoxic effect.

2. Use according to Claim 1, wherein the layer is furthermore used for the defined (i) improvement or (ii) reduction in the adhesion of biomolecules and/or cells (prokaryotes or eukaryotes).

3. Use of a cross-linked silicon-containing layer containing, consisting substantially of or consisting of silicon, O, C, H, optionally N, which can be produced by plasma polymerisation and/or cross-linking of organo-silicon liquids with the aid of a plasma process and/or UV radiation of a wavelength of less than 250 nm without the use of metals of an atomic number above 14, with an atomic ratio of oxygen to silicon of 0.75 to 2.2 and an atomic ratio of carbon to silicon of 0.1 to 2.5, measured with XPS, for the defined (i) improvement or (ii) reduction in the adhesion of biomolecules and/or cells (prokaryotes or eukaryotes), or
use according to Claim 2,
in each case with the proviso that, in the event that a reduction in the adhesion of biomolecules and/or cells takes place by virtue of the use, the cross-linked silicon-containing layer does not consist of carbon, silicon, oxygen and hydrogen and also optionally conventional impurities, wherein in the ESCA spectrum of the layer, with calibration to the aliphatic portion of the C 1s peak at 285.00 eV, in comparison with a trimethylsiloxy-terminated polydimethylsiloxane (PDMS) with a kinematic viscosity of 350 mm²/s at 25°C and a density of 0.97 g/ml at 25°C,
the Si 2p peak has a binding energy value that is shifted by at most 0.45 eV to higher or lower bond energies, and
the O 1s peak has a binding energy value that is shifted by at most 0.50 eV to higher or lower bond energies.

4. A non-genotoxic medical object, comprising a surface region with a cross-linked silicon-containing layer containing, consisting substantially of or consisting of silicon, O, C, H, optionally N, which can be produced by plasma polymerisation and/or cross-linking of organo-silicon liquids with the aid of a plasma process and/or UV radiation of a wavelength of less than 250 nm without the use of metals of an atomic number above 14, with an atomic ratio of oxygen to silicon of 0.75 to 2.2 and an atomic ratio of carbon to silicon of 0.1 to 2.5, measured with XPS, wherein the object is selected from the group consisting of:
a) membrane, pipe and tube, in particular oxygenator membrane, catheter, angioplasty balloon, stent, cannula, sensor and probe,
b) an object implanted when used as intended, selected from the group consisting of:
medical nails, clamps, threads and screws, in particular bone fixation nails, stents or vascular prostheses, injection systems, catheters, cardiovascular implants, artificial organs, in particular cardiac pacemakers and power source therefor, prostheses, orthopaedic implants, in particular artificial joint bodies, in particular sockets and counterparts which cooperate therewith, such as hip or knee prostheses, spinal prostheses, cochlear implants, artificial heart valves, heart valve rings or intraocular lenses, artificial corneas, pumps or other devices for releasing substances in the body and epithesis,
c) a container for holding and/or transporting body fluid, tissue or the constituents thereof of a living being, or biomolecules, preferably peptides, proteins, lipids, carbohydrates, nucleic acids or active substances produced therewith,
d) an object for at least partially covering skin or mucous membranes of a living being, and preferably wounds,
e) an object which when used as intended is otherwise in contact with body fluid, tissue or the constituents thereof of a living being, or with biomolecules, preferably peptides, proteins, lipids, carbohydrates, nucleic acids or active substances produced therewith,
**characterised in that** the carbon atoms of the silicon-containing layer have a proportion of 5 - 35% of carbon atoms with a bond to one oxygen atom ("C-O carbon"), measured with XPS, and/or
**in that** the carbon atoms of the silicon-containing layer have a proportion of 5 - 20% of carbon atoms with bonds to two oxygen atoms ("COO carbon"), measured with XPS.

5. An object according to Claim 4, **characterised in that** the object has a region of the silicon-containing layer with
a) a water contact angle of no more than 35°, or
b) a water contact angle of at least 90°.

6. An object according to one of Claims 4 to 5, **characterised in that** the silicon-containing layer, apart from hydrogen, consists of at least 98 atom % of the elements silicon, carbon and oxygen, and preferably of 0.1 % to 2% nitrogen, measured with XPS.

7. An object according to Claim 6, **characterised in that** the silicon-containing layer has 3-15 atom % carbon, relative to all the elements apart from hydrogen, determined by XPS.

8. An object according to one of Claims 4 to 7, **characterised in that** the silicon-containing layer is produced or can be produced by cross-linking a methylsiloxane precursor, preferably of hexamethyldisiloxane, by plasma polymerisation, in particular low-pressure or atmospheric-pressure plasma polymerisation, or by cross-linking a silicone oil without chemically reactive groups under the action of a plasma or UV radiation of a wavelength of less than 250 nm, in particular excimer radiation.

9. An object according to Claim 8, **characterised in that** the silicon-containing layer after the cross-linking of the precursor or silicone oil had been at least partially oxidised, preferably by the action of plasma, flame treatment, oxyfluorination, laser treatment or treatment with excimer lamps.

10. An object according to one of Claims 4 to 9, **characterised in that** the silicon-containing layer has a layer thickness of at most 2 µm, preferably at most 1 µm, particularly preferably at most 500 nm, and also at least 5 nm, preferably at least 10 nm, particularly preferably at least 15 nm.

11. A method for producing an object according to one of Claims 4 to 10, comprising the steps
a) provision of a cross-linked silicon-containing layer with an atomic ratio of oxygen to silicon of 0.75 to 2.2, and an atomic ratio of carbon to silicon of 0,1 to 2.5, measured with XPS, and
b) at least in sections, oxidation of the layer provided in step a).

12. A method according to Claim 11, **characterised in that** for limiting the region to be oxidised a mask, preferably a detachable self-adhesive mask, particularly preferably an adhesive strip, or a printed mask and/or a substance which is preferably at least partially soluble or dispersible in water is used and is preferably removed after step b).

13. Use according to one of Claims 1 to 3 for, compared with a corresponding, non-coated surface,
- reducing the adhesion of pathogenic deposits, and/or
- reducing thrombogenicity, and/or
- reducing the occurrence of humoral and cellular immune reactions, and/or
- reducing non-specific adsorption of peptides, proteins, lipids, carbohydrates and/or nucleic acids on the surface, and/or
- permitting spatially limited growth of cell cultures on the surface, and/or
- increasing wettability by aqueous liquids, in particular blood.

## Revendications

1. Utilisation d'une couche siliciée réticulée, sensiblement composée de ou composée de silicium, O, C, H, optionnellement N, qui peut être fabriquée par polymérisation plasma et/ou réticulation de liquides organiques siliciés à l'aide d'un processus au plasma et/ou d'un rayonnement UV d'une longueur d'onde inférieure à 250 nm sans l'utilisation de métaux d'un nombre atomique supérieur à 14, avec un rapport atomique oxygène sur silicium de 0,75 à 2,2 et un rapport atomique carbone sur silicium de 0,1 à 2,5, mesurés par XPS,
- comme surface non génotoxique , ou
- pour soumettre une surface à une action non génotoxique ou doter une surface à une action non génotoxique.

2. Utilisation selon la revendication 1, la couche étant en outre utilisée pour améliorer (i) ou réduire (ii) de façon définie l'adhérence de biomolécules et/ou de cellules (procaryotes ou eucaryotes).

3. Utilisation d'une couche siliciée réticulée qui, sensiblement composée de ou composée de silicium, O, C, H, optionnellement N, qui peut être fabriquée par polymérisation plasma et/ou réticulation de liquides organiques siliciés à l'aide d'un processus au plasma et/ou d'un rayonnement UV d'une longueur d'onde inférieure à 250 nm sans l'utilisation de métaux d'un nombre atomique supérieur à 14, avec un rapport atomique oxygène sur silicium de 0,75 à 2,2 et un rapport atomique carbone sur silicium de 0,1 à 2,5, mesurés par XPS, pour améliorer (i) ou réduire (ii) de façon définie l'adhérence de biomolécules et/ou de cellules (procaryotes ou eucaryotes) ou utilisation selon la revendication 2,
à chaque fois avec la consigne que, dans le cas où une réduction de l'adhérence de biomolécules et/ou de cellules est effectuée par l'utilisation, la couche siliciée réticulée n'est pas composée de carbone, de silicium, d'oxygène et d'hydrogène ainsi que éventuellement d'impuretés habituelles ; et dans le spectre ESCA de la couche, lors de l'étalonnage sur la partie aliphatique du pic C 1s à 285,00 eV, en comparaison avec un polydiméthylsiloxane (PDMS) à terminaison triméthylsiloxy d'une viscosité cinématique de 350 mm²/s à 25°C et d'une densité de 0,97 g/ml à 25°C,
le pic Si 2p a une énergie de liaison d'une valeur qui est décalée de 0,45 eV maximum par rapport à des énergies de liaison plus élevées ou plus basses, et
le pic O 1s a une énergie de liaison d'une valeur qui est décalée de 0,50 eV maximum par rapport à des énergies de liaison plus élevées ou plus basses.

4. Objet non génotoxique pour la technique médicale, présentant une région de surface dotée d'une couche siliciée réticulée, sensiblement composée de ou composée de silicium, O, C, H, optionnellement N, qui peut être fabriquée par polymérisation plasma et/ou réticulation de liquides organiques siliciés à l'aide d'un processus au plasma et/ou d'un rayonnement UV d'une longueur d'onde inférieure à 250 nm sans l'utilisation de métaux d'un nombre atomique supérieur à 14, avec un rapport atomique oxygène sur silicium de 0,75 à 2,2 et un rapport atomique carbone sur silicium de 0,1 à 2,5, mesurés par XPS, l'objet étant sélectionné dans le groupe constitués par :
a) une membrane, un tube et un tuyau, notamment une membrane d'oxygénation, un cathéter, un ballon d'angioplastie, un stents, une canule, un capteur et une sonde,
b) un objet implanté pour un usage déterminé et choisi dans le groupe constitué par : un clou, une agrafe, un fil et une vis à usage médical, notamment un clou de fixation osseuse, un stent ou une prothèse de vaisseau, un système d'injection, un cathéter, un implant cardiovasculaire, un organe artificiel, notamment un stimulateur cardiaque et sa source de courant, une prothèse, un implant orthopédique, notamment un corps articulé artificiel, notamment une cavité glénoïde et une pièce homologue coopérant avec celle-ci, comme une prothèse du genou et de la hanche, une prothèse de colonne vertébrale, un implant cochléaire, une valvule cardiaque artificielle, une valvule cardiaque cylindrique ou une lentille intraoculaire, une cornée artificielle, une pompe ou autre dispositif de libération de substances dans le corps et une épithèse,
c) un récipient destiné à recevoir et/ou transporter un fluide corporel, un tissu et ses constituants d'un être vivant ou des biomolécules, de préférence des peptides, des protéines, des lipides, des hydrates de carbone, des acides nucléiques ou des substances actives fabriquées avec ceux-ci,
d) un objet destiné à recouvrir au moins partiellement la peau ou une muqueuse d'un être vivant et de préférence des blessures,
e) un objet en contact d'autre part pour un usage déterminé avec un liquide corporel, un tissu ou ses constituants d'un être vivant ou avec des biomolécules, de préférence des peptides, des protéines, des lipides, des hydrates de carbone, des acides nucléiques ou des substances actives fabriquées avec ceux-ci,
**caractérisé en ce que** les atomes de carbone de la couche siliciée ont un pourcentage de 5 à 35% d'atomes de carbone présentant une liaison avec un atome d'oxygène (« C-O-carbone »), mesuré par XPS et/ou
**en ce que** les atomes de carbone de la couche siliciée ont un pourcentage de 5 à 20% d'atomes de carbone présentant une liaison avec deux atomes d'oxygène (« COO-carbone »), mesuré par XPS.

5. Objet selon la revendication 4, **caractérisé en ce que** l'objet possède une région de la couche siliciée ayant
a) un angle de contact de l'eau non inférieur à 35°, ou
b) un angle de contact de l'eau d'au moins 90°.

6. Objet selon l'une des revendications 4 à 5, **caractérisé en ce que**, exception faite de l'hydrogène, la couche siliciée a un pourcentage d'atomes d'éléments silicium, carbone et oxygène d'au moins 98% et de préférence un pourcentage d'azote de 0,1% à 2%, mesurés par XPS.

7. Objet selon la revendication 6, **caractérisé en ce que** la couche siliciée a un pourcentage d'atomes de carbone de 3 à 15% par rapport à tous les éléments, exception faite de l'oxygène, déterminé par XPS.

8. Objet selon l'une des revendications 4 à 7, **caractérisé en ce que** la couche siliciée est fabriquée ou peut être fabriquée par réticulation d'un précurseur méthylsiloxane, de préférence à partir d'hexaméthyldisiloxane par polymérisation plasma, notamment par polymérisation plasma à basse pression ou à la pression atmosphérique ou par réticulation d'une huile de silicone sans groupes réactifs chimiques par action d'un plasma ou d'un rayonnement UV d'une longueur d'onde inférieure à 250 nm, notamment par rayonnement à excimères.

9. Objet selon la revendication 8, **caractérisé en ce que** après la réticulation du précurseur ou de l'huile de silicone la couche siliciée a été au moins partiellement oxydée, de préférence par action d'un plasma, application de flamme, oxy-fluoration, traitement laser ou traitement avec des lampes à excimères.

10. Objet selon l'une des revendications 4 à 9, **caractérisé en ce que** la couche siliciée a une épaisseur de couche de 2 µm au plus, de préférence de 1 µm au plus, de façon particulièrement préférée de 500 nm au plus, ainsi que de 5 nm au moins, de préférence de 10 nm au moins, de façon particulièrement préférée de 15 nm au moins.

11. Procédé de fabrication d'un objet selon l'une des revendications 4 à 10, comportant les étapes
a) de préparation d'une couche siliciée réticulée avec un rapport atomique oxygène sur silicium de 0,75 à 2,2 et un rapport atomique carbone sur silicium de 0,1 à 2,5, mesurés par XPS, et
b) d'oxydation au moins par portions de la couche préparée à l'étape a).

12. Procédé selon la revendication 11, **caractérisé en ce que** pour limiter la région à oxyder on utilise un masque, de préférence un masque autocollant détachable, de façon particulièrement préférée une bande adhésive, ou un masque imprimé et/ou une matière qui peut être au moins partiellement dispersée ou dissoute de préférence dans l'eau et on retire de préférence ledit masque après l'étape b).

13. Utilisation selon l'une des revendications 1 à 3, par comparaison avec une surface correspondante sans revêtement, pour
- réduire une adhérence de dépôts pathogènes et/ou
- réduire la thrombogénicité et/ou
- réduire la survenance d'immunoréactions humorales et cellulaires et/ou
- réduire l'adsorption non spécifique de peptides, de protéines, de lipides, d'hydrates de carbone et/ou d'acides nucléiques sur la surface et/ou
- permettre une croissance, limitée dans l'espace, de cultures cellulaires sur la surface et/ou
- augmenter la mouillabilité par des liquides aqueux, notamment du sang.
